(19) Europäisches Patentamt
European Patent Office
Office européen des brevets

(11) Numéro de publication: **0 305 297**
**B1**

(12) # FASCICULE DE BREVET EUROPEEN

(45) Date de publication du fascicule du brevet: **16.05.90**

(51) Int. Cl.⁵: **C07K 5/06,** A61K 37/02

(21) Numéro de dépôt: **88402165.0**

(22) Date de dépôt: **26.08.88**

(54) **Nouveaux dérivés de l'acide aza - 2 bicyclooctane carboxylique - 3, leurs procédés de préparation et les compositions pharmaceutiques qui les contiennent.**

(30) Priorité: **28.08.87 FR 8712013**

(43) Date de publication de la demande: **01.03.89 Bulletin 89/9**

(45) Mention de la délivrance du brevet: **16.05.90 Bulletin 90/20**

(84) Etats contractants désignés: **AT BE CH DE ES FR GB GR IT LI LU NL SE**

(56) Documents cités:
**EP-A- 0 051 020**
**EP-A- 0 105 102**

**Le dossier contient des informations techniques présentées postérieurement au dépôt de la demande et ne figurant pas dans le présent fascicule.**

(73) Titulaire: **ADIR ET COMPAGNIE, 22, rue Garnier, F-92201 Neuilly sur Seine(FR)**

(72) Inventeur: **Vincent, Michel, 8 allée du Prunier Hardy, F-92220 Bagneux(FR)**
Inventeur: **Remond, Georges, 9 avenue des Etats-Unis, F-78000 Versailles(FR)**
Inventeur: **Laubie, Michel, 35 avenue Foch, F-92420 Vaucresson(FR)**

**EP 0 305 297 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'acide aza - 2 bicyclooctane carboxylique 3, leurs modes de préparation et les compositions pharmaceutiques qui les contiennent.

Plus spécifiquement, la présente invention concerne les dérivés de formule (I):

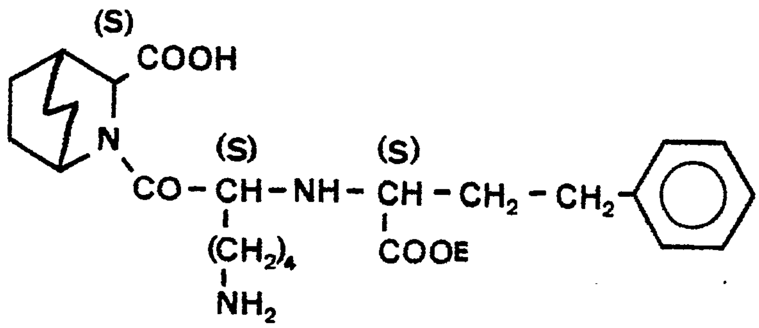

(I)

dans laquelle E signifie un groupement alkyle inférieur ou, préférentiellement un atome d'hydrogène, ainsi que leurs sels d'addition à un acide ou une base pharmaceutiquement acceptables. La demande de brevet européen no 0 105 102 décrit le [αN-(carboxy-1(S) cyclohexyl-3propyl)-(5)lysyl]-2 carboxyl-3(S) aza-2 bicyclo - [2,2,2] octane et le [αN-(ethoxycarbonyl-1(S) cyclohexyl-3 propyl)-(S)lysyl]-2carboxyl-3(S) aza-2 bicyclo-[2,2,2]octane chlorhydrate, sans mentionner leur activité biologique.

On connaît le brevet européen no 0 051 020 qui revendique des composés de formule générale (II):

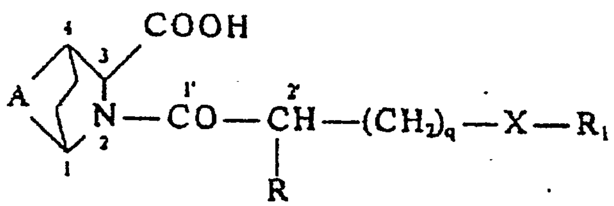

(II)

dans laquelle A représente un radical vinylène ou diméthylène,

q est 0 ou 1,

R représente un radical alkyle inférieur pouvant porter un groupe amino,

X représente - S - et $R_1$ représente H, ou bien X représente - NH - et $R_1$ représente un atome d'hydrogène ou un radical de formule :

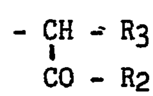

où $R_2$ représente un hydroxyle ou un groupement alcoxy inférieur

et $R_3$ représente en particulier un atome d'hydrogène, un radical alkyle linéaire ou ramifié, cycloalkyle ou phénylalkyle, ayant au plus et au total 8 atomes de carbone,

les expressions radicaux alkyle ou alcoxy inférieurs signifiant des groupes ayant de 1 à 4 atomes de carbone.

Les dérivés préférés du brevet européen n° 0 051 020 ont pour valeur de R le groupement méthyle (page 2, ligne 58).

Les dérivés de formule (I) sont donc des cas particuliers des dérivés de formule (II) pour lesquels R représente un groupement amino - 4 butyle. Les dérivés de formule (I) ne sont pas les dérivés préférés du brevet européen n° 0 051 020.

La demanderesse a, en effet, découvert que le dérivé de formule (I) pour lequel E représente un atome d'hydrogène possède une activité inhibitrice de l'enzyme de conversion de l'angiotensine I en angiotensine II dont le niveau et la durée d'action sont surprenants et nettement supérieurs à ceux des dérivés préférés du brevet européen n° 0 051 020. Ainsi, lorsqu'il est administré par voie orale à une dose de 1 $mg.kg^{-1}$, le dérivé de formule (I) pour lequel E = H provoque très rapidement après son administration un pourcentage d'inhibition de l'enzyme de conversion très élevé (80 %, 15 minutes après administration, voisin de 100 %, 30 minutes seulement après son administration), un très haut niveau d'activité étant maintenu longtemps après l'administration puisque le pourcentage d'inhibition de l'enzyme de conversion reste supérieur à 70 %, 6 heures après l'administration. Une telle dose par voie orale d'un des

composés du brevet européen n° 0 051 020 ne permet pas d'obtenir une activité qui soit à la fois aussi intense, rapide et de longue durée.

L'intensité de l'activité par voie orale que possède le composé de la présente invention pour lequel E est un atome d'hydrogène est d'autant plus inattendue que ce composé possède une structure diacide.

En effet, les composés spécifiquement revendiqués dans le brevet européen n° 0 051 020 pour lesquels X est NH, ont une structure d'ester au niveau de la chaîne latérale (revendications 6 et 7).

La structure diacide effectivement, est en général défavorable à une bonne absorption par voie orale ; les dérivés de structure diacide ont donc une activité thérapeutique de faible intensité après administration par cette voie. Ces dérivés sont donc généralement réservés à l'administration par voie intraveineuse où leur rapidité d'action est intéressante.

Par ailleurs, il est connu que l'estérification de la fonction acide carboxylique de la chaîne latérale améliore la lipophilie de la molécule, et donc, en facilite l'absorption après administration par voie orale. Toutefois, cette fonction ester doit alors être hydrolysée dans l'organisme vivant, par des enzymes spécifiques, pour pouvoir libérer le diacide qui seul possède une efficacité thérapeutique. Ceci explique, que généralement chez les inhibiteurs de l'enzyme de conversion de l'art antérieur - et plus particulièrement chez les dérivés du brevet n° 0 051 020 - destinés à l'administration par voie orale, la fonction acide carboxylique est estérifiée.

Toutefois, cette structure d'ester présente un inconvénient : le temps nécessaire à l'organisme pour hydrolyser la fonction ester de la chaîne latérale en acide retarde l'apparition d'une activité intense.

Avec le composé de la présente pour lequel E est un atome d'hydrogène, les inconvénients inhérents à la structure diacide ainsi qu'à la structure ester ont disparu, pour ne laisser place qu'aux avantages que possèdent ces deux structures. En effet, le dérivé de la présente invention pour lequel E = H, est particulièrement bien absorbé après administration par voie orale comme en témoigne l'intensité de son action après administration par cette voie. En outre, ce composé a conservé les avantages de la structure diacide puisque très peu de temps après son administration, il présente une intense (80 % d'inhibition de l'enzyme de conversion 15 minutes seulement après son administration).

Ces facteurs font que le composé de la présente invention pour lequel E est un atome d'hydrogène se démarque très nettement des composés de l'art antérieur pour son utilisation en thérapeutique.

Toutefois, l'invention se rapporte également aux composés pour lesquels E est un radical alkyle inférieur puisque, en vertu de ce qui précède, ces composés sont des prodrugs du composé pour lesquels E est un atome d'hydrogène.

L'invention se rapporte également aux sels d'addition des composés de formule (I) obtenus avec une base ou un acide minéral ou organique thérapeutiquement compatibles.

Parmi les acides que l'on peut ajouter aux composés de formule (I) pour former un sel d'addition, on peut citer, à titre d'exemple, les acides chlorhydrique, sulfurique, phosphorique, tartrique, malique, maléique, fumarique, oxalique, méthane-sulfonique, éthane-sulfonique, camphorique, citrique, etc...

Comme bases pouvant salifier les composés de formule (I) on pourra utiliser des hydroxydes de sodium, potassium, calcium ou d'aluminium, des carbonates de métaux alcalins ou alcalinoterreux ou des bases organiques comme la triéthylamine, la benzylamine, la diéthanolamine, la tert.butylamine, la dicyclohexylamine, l'arginine, etc...

Les composés de formule (I) ainsi que leurs sels possèdent des propriétés pharmacologiques intéressantes. Ils exercent notamment une activité inhibitrice sur certaines enzymes, comme les carboxypolypeptidases, les enképhalinases ou la kininase II. Ils inhibent, en particulier, la transformation du décapeptide angiotensine I en l'octapeptide angiotensine II, responsable dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque. L'action sur la kininase II a pour résultat une augmentation de la bradykinine circulante et également une baisse de la tension artérielle.

Les composés de la présente invention sont, non seulement utiles dans le traitement de l'hypertension, mais également utilisables pour soigner l'insuffisance cardiaque aigüe et chronique, dans le traitement de l'hyperaldosteronisme secondaire, dans le traitement de l'hypertension pulmonaire primaire et secondaire, dans le glaucome, dans l'insuffisance rénale, dans l'hypertension vasculaire rénale, dans le traitement des troubles vasculaires périphériques comme la migraine, la maladie de Raynaud, l'athérosclérose, les thromboses, l'artérite des membres inférieures, les cirrhoses, ascites. Par ailleurs, les composés de la présente invention possèdent également une activité inhibitrice de l'enképhalinase et peuvent donc avoir une utilisation en tant qu'analgésique ou antiinflammatoire.

L'invention s'étend aussi aux compositions pharmaceutiques renfermant comme principe actif un composé de formule générale (I) ou un de ses sels d'addition, avec une base ou un acide minéral ou organique, en association avec un ou plusieurs excipients inertes, non toxiques convenant pour l'usage pharmaceutique et/ou un agent liant, un agent aromatisant, un agent de délitement, un agent édulcorant, un agent lubrifiant ou bien encore un véhicule liquide adapté à l'administration par voie intraveineuse, tel que l'eau stérile apyrogène.

Parmi les compositions pharmaceutiques selon l'invention, on pourra citer, plus particulièrement celles qui conviennent pour l'administration orale, parentérale, oculaire, per ou transcutanée, nasale, rectale

perlinguale, ou respiratoire et notamment les préparations injectables, les aérosols, les gouttes oculaires ou nasales, les comprimés, les comprimés sublinguaux, les gélules, capsules, tablettes, glossettes, suppositoires, crèmes, pommades et gels.

Les compositions pharmaceutiques selon l'invention peuvent en outre contenir un autre principe actif d'action synergique ou complémentaire.

Parmi ces derniers principes actifs, on pourra citer un diurétique et, notamment, un saliurétique, comme par exemple un thiazide, un dihydrothiazide, un chlorosulfamide, un acide dihydrobenzofuranne carboxylique - 2 ou un dérivé de l'acide phénoxy acétique. Des exemples de tels composés sont la N - (chloro - 3′ sulfamoyl - 4′benzamido) méthyl -2 indoline, l'acide éthacrynique, le furosémide.

On pourra, également, ajouter une substance α- adrénolytique, un β bloquant, un antagoniste calcique ou un agoniste des récepteurs dopaminergiques vasculaires.

La posologie utile peut varier largement en fonction de l'âge, du poids du patient, de la sévérité de l'indication thérapeutique ainsi que la voie d'administration. La voie d'administration préférée est la voie buccale mais la voie intraveineuse est également parfaitement appropriée au traitement de l'hypertension. D'une manière générale, la posologie unitaire s'échelonnera de préférence entre 0,05 et 20 mg.

L'invention se rapporte également aux procédés d'obtention des composés de formule (I) caractérisés en ce que l'on soumet un dérivé de formule (III) :

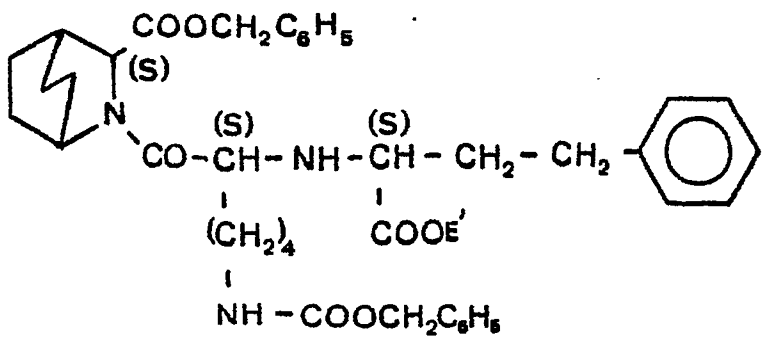

(III)

dans lequel E′ représente un groupement alkyle inférieur ou benzyle, à hydrogénation en présence d'un catalyseur tel que le charbon palladié pour obtenir :

- le composé de formule (I) pour lequel E = H dans le cas où E′ représente un groupement benzyle,
- un composé de formule (I) pour lequel E est un groupement alkyle inférieur dans le cas où E′ représente un groupement alkyle inférieur, composé qui peut éventuellement être soumis à hydrolyse alcaline pour obtenir le composé de formule (I) pour lequel E = H,

le composé de formule (I) ainsi obtenu, si on le désire, pouvant être salifié par un acide ou une base minéral ou organique thérapeutiquement compatible :

le composé de formule (III) étant lui-même obtenu :

- soit en soumettant à amination réductrice un oxo - 2 phényl - 4 butyrate de formule (IV) :

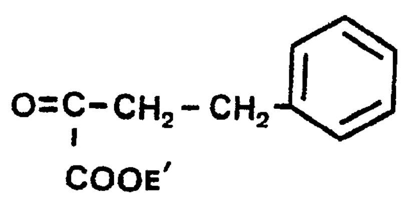

(IV)

ou E′ a la même signification que dans la formule (III),

en présence d'un hydrure mixte de métal alcalin ou d'un cyanohydrure mixte de métal alcalin tel que le cyanoborohydrure de sodium,

avec un dérivé de la lysine, dans lequel les groupements εaminé et carboxylique sont protégés par des groupements appropriés, tel que le εN benzyloxycarbonyl (S) lysinate de tert.butyle de formule (V) :

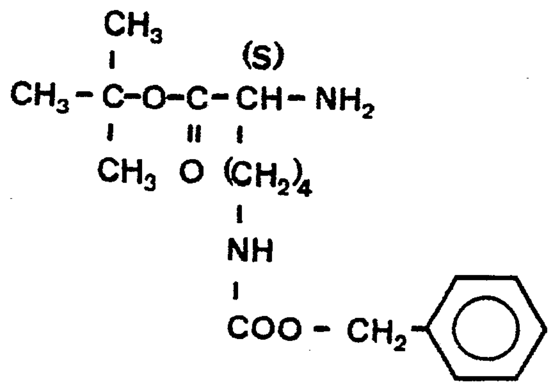

(V)

pour obtenir un dérivé de formule (VI) :

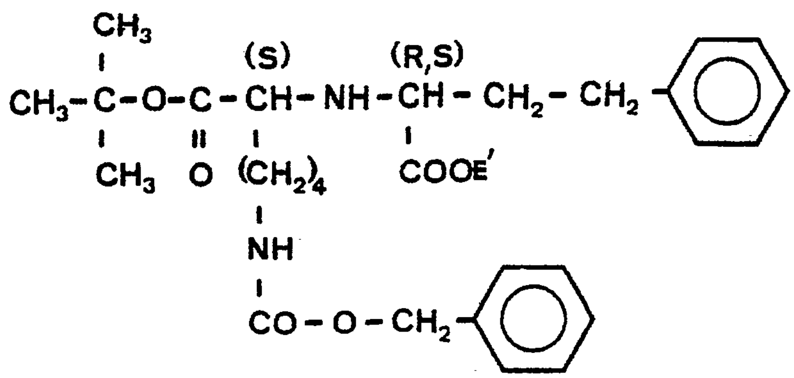

(VI)

dans lequel E′ a la même signification que dans la formule (III),
dont on isole les isomères (S, R) et (S, S) par une technique usuelle de séparation comme la chromatographie sur colonne de silice,
l'isomère (S, S) ainsi obtenu étant alors soumis à une déprotection en milieu acide de la fonction acide carboxylique de la lysine pour obtenir un dérivé de formule (VII) :

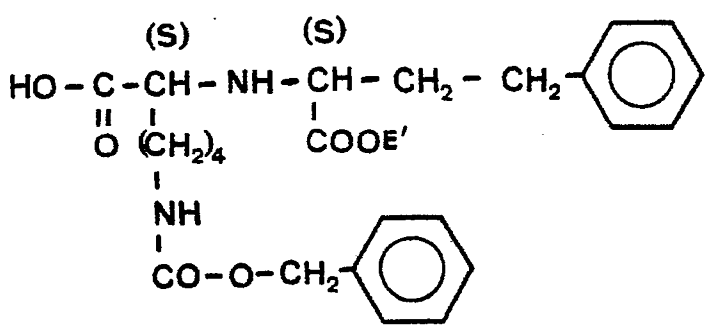

(VII)

dans lequel E′ a la même signification que dans la formule (III),
qui est alors condensé avec un ester de l'acide aza - 2 bicyclooctane [2,2,2] carboxylique - 3 (S) et préférentiellement avec l'ester benzylique de formule (VIII) :

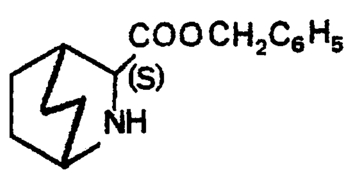

(VIII)

en présence d'un agent de couplage peptidique tel que le dicyclohexycarbodiimide en présence d'hydroxybenzotriazole,
pour obtenir un dérivé de formule (III),
- soit par couplage d'un ester de l'acide aza - 2 bicyclooctane [2,2,2] carboxylique - 3 (S) et préférentiellement de l'ester benzylique de formule ci-dessus référencée (VIII), avec un dérivé de la lysine dans lequel les groupements αet εaminés sont protégés par des groupements appropriés tel que l'αN tert.butoxycarbonyl εN benzyloxycarbonyl (S) lysine de formule (IX) :

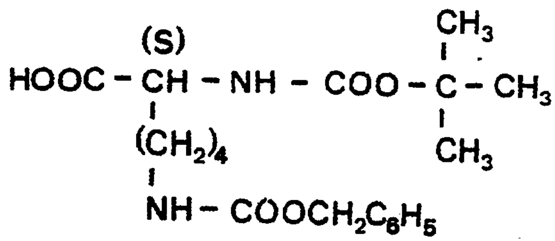

(IX)

en présence d'un agent de couplage peptidique tel que le dicyclohexylcarbodiimide en présence d'hydroxybenzotriazole,
pour obtenir un dérivé de formule (X) :

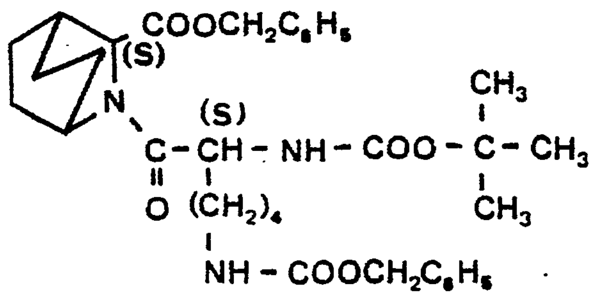

(X)

que l'on soumet à une déprotection en milieu acide de la fonction αaminée du reste lysyle,
pour obtenir un dérivé de formule (XI) :

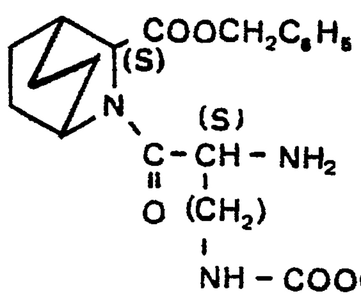

(XI)

que l'on soumet à amination réductrice en présence d'un hydrure mixte de métal alcalin ou d'un cyanohydrure mixte de métal alcalin tel que le cyanoborohydrure de sodium,
avec un dérivé de formule (IV) ci-dessus référencé,
pour obtenir le dérivé de formule (III) sous forme de racémique (III, R, S) :

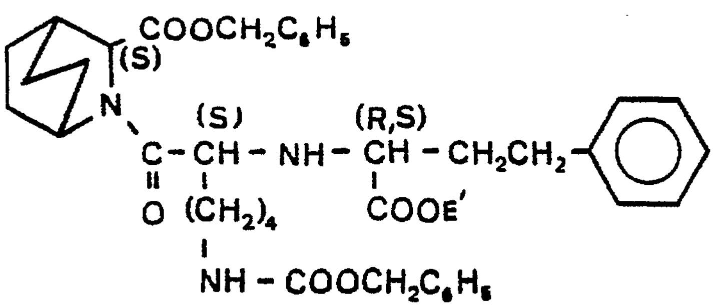

(III, R, S)

dans lequel E' a la même signification que dans la formule (III), dont on isole l'isomère (S, S, S) par une technique usuelle de séparation comme la chromatographie sur colonne de silice.

Les composés de formule (III) et leur racémique de formule (III, R, S), ainsi que leurs sels d'addtion à un acide minéral ou organique sont nouveaux et font partie de l'invention au même titre que les composés de formule (I).

Les exemples suivants illustrent l'invention et ne la limitent en aucune façon.

Les produits de départ sont connus de la littérature.

**EXEMPLE 1 : CHLORHYDRATE DE [N - (CARBOXY - 1 (S) PHENYL - 3 PROPYL - (S) LYSYL] - 2 CARBOXY - 3 (S) AZA - 2 BICYCLO - [2,2,2] OCTANE**

**STADE A :**

αN-[carbéthoxy - 1 (S) phényl - 3 propyl] εN benzyloxycarbonyl (S) lysinate de tert.butyle

Dissoudre 10 g (0,0268 mol) de chlorhydrate de εN-benzyloxycarbonyl lysinate de tert.butyle dans 150 $cm^3$ d'éthanol anhydre ; ajouter 2,71 g (0,0268 mol) de triéthylamine, 20,6 g (0,1 mol) d'oxo - 2 phényl - 4 butyrate d'éthyle et 20 g de tamis moléculaire 0,4 mm (4Å). Agiter une heure à 20°C. Additionner ensuite en 4 heures une solution de 1,68 g (0,0268 mol) de cyanoborohydrure de sodium dans 20 $cm^3$ d'éthanol, puis continuer l'agitation 18 heures à 20°C. Filtrer et amener à sec, redissoudre dans 200 $cm^3$ d'acétate d'éthyle, laver deux fois par 100 $cm^3$ d'une solution aqueuse d'acide citrique à 10 %, puis à l'eau jusqu'à neutralité des eaux de lavage. Sécher sur sulfate de magnésium et évaporer. On recueille 28,7 g d'une huile qui est purifiée par chromatographie sur gel de silice (éluant : chlorure de méthylène : 95, acétone : 5). Après élimination d'impuretés puis de l'isomère (S, R) on recueille 4,1 g de α[N-(carbéthoxy - 1 (S) phényl - 3 propyl] εN-benzyloxycarbonyl (S) lysinate de tert.butyle qui sont utilisés tels quels au stade suivant.

STADE B :

Chlorhydrate de αN-[carbéthoxy - 1 (S) phényl - 3 propyl] εN-benzyloxycarbonyl (S) lysine

Dissoudre 4,1 g de αN-[carbéthoxy - 1 (S) phényl - 3 propyl] εN benzyloxycarbonyl (S) lysinate de tert.butyle obtenus au stade précédent dans 100 $cm^3$ d'une solution 4N d'acide chlorhydrique dans le dioxanne, et abandonner 24 heures la solution à 20°C. Amener à sec et concrétiser dans l'éther. Après séchage au dessicateur, on récupère 3,8 g de chlorhydrate de αN[carbéthoxy - 1 (S) phényl - 3 propyl] εN-benzyloxycarbonyl (S) lysine.

| Composition centésimale: | | | | |
|---|---|---|---|---|
| Calculée: | C : 61,59 | H : 6,96 | N : 5,52 | Cl : 6,99 |
| Trouvée: | C : 61,34 | H : 7,26 | N : 5,63 | Cl : 6,93 |

Spectographie infra rouge : vs NH : 3360 $cm^{-1}$
vs CO : 1750 et 1690 $cm^{-1}$.

**STADE C :**

[αN-(carbéthoxy - 1(S) phényl - 3 propyl) εN benzyloxycarbonyl (S) lysyl] - 2 benzyloxycarbonyl - 3(S) aza - 2 bicyclo [2,2,2] octane

Le chlorhydrate de αN-[carbéthoxy - 1 (S) phényl - 3 propyl] εN-benzyloxycarbonyl (S) lysine obtenu au stade précédent est couplé selon la technique de W. KÖNIG, R. GEIGER - Chem. Ber. (1970), 103, 788 avec l'ester benzylique de l'acide aza - 2 bicyclo [2,2,2] octane carboxylique - 3 (S) décrit dans le brevet européen n° 0 051 020.

Le [αN-(carbéthoxy - 1(S) phényl - 3 propyl) εN-benzyloxycarbonyl (S) lysyl] - 2 benzyloxycarbonyl - 3 (S) aza - 2 bicyclo [2,2,2] octane obtenu, purifié par chromatographie sur gel de silice (éluant : acétate d'éthyle : 50, cyclohexane : 50) est utilisé tel quel au stade suivant.

**STADE D :**

Dichlorhydrate de [αN-(carbéthoxy - 1 (S) phényl - 3 propyl) - (S) lysyl] - 2 carboxy - 3 (S) aza - 2 bicyclo [2,2,2] octane

Dissoudre 2,8 g de [αN-(carbéthoxy - 1 (S) phényl - 3 propyl) εN benzyloxycarbonyl (S) lysyl] - 2 benzyloxycarbonyl - 3 (S) aza - 2 bicyclo [2,2,2] octane obtenu au stade précédent dans 100 $cm^3$ d'éthanol ; ajouter 0,2 g de charbon palladié à 10 % et hydrogéner pendant 18 heures à 20°C sous pression d'hydrogène de 3 $kg/cm^2$.

Filtrer le catalyseur, ajouter 10 $cm^3$ d'une solution 4N d'acide chlorhydrique dans l'éther et amener à sec. Reprendre par l'acétate d'éthyle, filtrer et sécher au dessicateur. On obtient 1,75 g (Rendement : 80 %) de dichlorhydrate de [αN-(carbéthoxy - 1 (S) phényl - 3 propyl) (S) lysyl] - 2 carboxy - 3 (S) aza - 2 bicyclo [2,2,2] octane.

| Composition centésimale: | | | | |
|---|---|---|---|---|
| Calculée: | C : 57,14 | H : 7,56 | N : 7,69 | Cl : 12,97 |
| Trouvé: | C : 56,76 | H : 7,31 | N : 7,76 | Cl : 12,97 |

Etude spectrale : Spectrométrie infra rouge νCO : 1650 et 1740 $cm^{-1}$

Spectrométrie de masse : Spectre DCI ($NH_3$)

M/Z : 474 : $[M + H]^{\oplus}$

456 : $[M + H - H_2O]^{\oplus}$

156 : 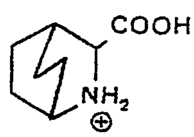

**STADE E :**

Dichlorhydrate de [αN-(carboxy - 1(S) phényl - 3 propyl) - (S) lysyl] - 2 carboxy - 3 (S) aza - 2 bicyclo [2,2,2] octane

1,75 g (2,75 mmol) de dichlorhydrate de [αN(carbéthoxy - 1(S) phényl - 3 propyl) - (S) lysyl] - 2 carboxy - 3 (S) aza - 2 bicyclo [2,2,2] octane obtenu au stade précédent est dissous dans 120 $cm^3$ (1,2 mmol) de soude 0,1 N. Après 48 heures à température ambiante la solution est neutralisée par 12 $cm^3$ d'acide chlorhydrique 1N puis amenée à sec. Redissoudre le résidu dans 30 $cm^3$ d'isopropanol anhydre et éliminer le chlorure de sodium insoluble par filtration. Amener à sec et renouveler cette opération. Reprendre par l'eau, filtrer sur filtre Millipore® 0,22 μm et lyophiliser. On recueille 1,2 g de dichlorhydrate de [αN-(carboxy - 1(S) phényl - 3 propyl) - (S) - lysyl] - 2 carboxy - 3 (S) aza - 2 bicyclo [2,2,2] octane.

Pouvoir rotatoire :

$[\alpha]_D^{21°C}$ = 18,2° (c : 1% pyridine)

Etude spectrale :

Spectrométrie infra-rouge : νCO acide 1710 $cm^{-1}$
νCO 1640 $cm^{-1}$

Spectrométrie de masse : DCI

M/Z : 446 : $[M + H]^{\oplus}$

428 : $[M + H - H_2O]^{\oplus}$

291 : 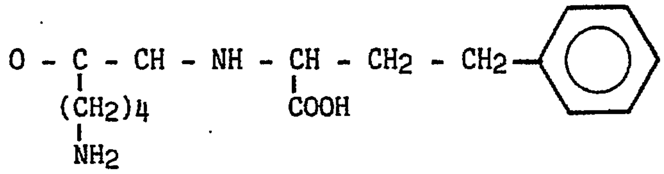

156 : 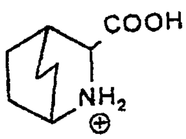

110 : 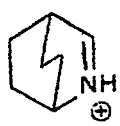

Spectrométrie de résonance magnétique nucléaire : Solvant $D_2O$ (T = 301° K)

$\delta^1H$ en ppm TMS (Référence externe)

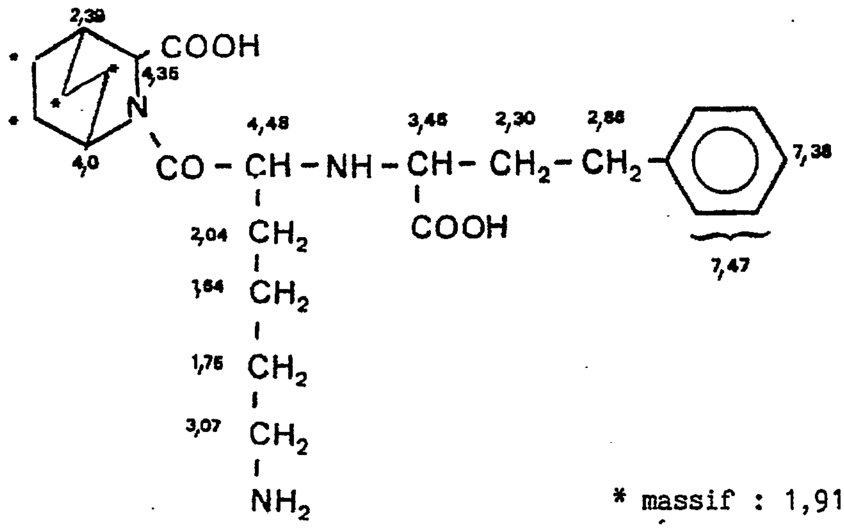

* massif : 1,91 à 1,64 ppm

$\delta^{13}C$ en ppm TMS (Référence externe)

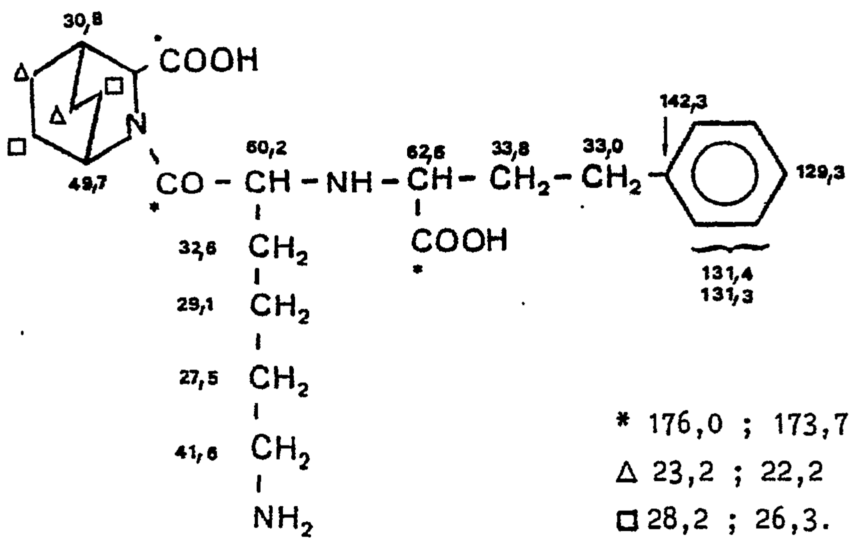

* 176,0 ; 173,7 ; 169,1

Δ 23,2 ; 22,2

□ 28,2 ; 26,3.

**EXEMPLE 2: [αN - (ETHOXYCARBONYL - 1 (S) PHENYL - 3 PROPYL) - (S) LYSYL] - 2 CARBOXYL - 3 (S) AZA - 2 BICYCLO - [2,2,2] OCTANE DICHLORHYDRATE**

**STADE A :**

(αN-tert.butoxycarbonyl εN-benzyloxycarbonyl (S) lysyl) - 2 benzyloxycarbonyl - 3 (S) aza - 2 bicyclo [2,2,2] octane

L'αN-tert.butoxycarbonyl εN-benzyloxycarbonyl (S) lysine est couplée selon la technique de W. KÖNIG, R. GEIGER - Chem. Ber. (1970), <u>103</u>, 788 avec l'ester benzylique de l'acide aza - 2 bicyclo [2,2,2] octane carboxylique - 3 (S) décrit dans le brevet européen n° 0 051 020.

L'(αN-tert.butoxycarbonyl εN-benzyloxycarbonyl (S) lysyl) - 2 benzyloxycarbonyl - 3 (S) aza - 2 bicyclo [2,2,2] octane obtenu, purifié par chromatographie sur gel de silice, est utilisé tel quel au stade suivant.

**STADE B :**

(εN-benzyloxycarbonyl - (S) lysyl) - 2 benzyloxycarbonyl - 3(S) aza - 2 bicyclo [2,2,2] octane

En utilisant la méthode de déprotection par l'acide trifluoroacétique dans le chlorure de méthylène anhydre décrite par B. GUTTE et K.B. MERRIFIELD (J.Am. Chem. Soc. 1969, 91, 501) on obtient quantitativement, à partir de l'(αN-tert.butoxycarbonyl εN benzyloxycarbonyl - (S) lysyl) - 2 benzyloxycarbonyl - 3 (S) aza - 2 bicyclo [2,2,2] octane préparé au stade précédent l'(εN-benzyloxycarbonyl - (S) lysyl) - 2 benzyloxycarbonyl - 3 (S) aza - 2 bicyclo [2,2,2] octane.

**STADE C :**

[αN-(carbéthoxy - 1 (S) phényl - 3 propyl) εN-benzyloxycarbonyl (S) lysyl] - 2 benzyloxycarbonyl - 3 (S) aza - 2 bicyclo [2,2,2] octane

Le (εN-benzyloxycarbonyl (S) lysyl] - 2 benzyloxycarbonyl - 3 (S) aza - 2 bicyclo [2,2,2] octane obtenu au stade précédent est soumis à amination réductrice en présence de cyanoborohydrure de sodium avec l'oxo - 2 phényl - 4 butyrate d'éthyle pour obtenir après purification sur gel de silice et élimination de l'isomère (S, S, R), le [αN-(carbéthoxy - 1 (S) phényl - 3 propyl) εN-benzyloxycarbonyl (S) lysyl] - 2 benzyloxycarbonyl - 3 (S) aza - 2 bicyclo [2,2,2] octane utilisé tel quel au stade suivant.

**STADE D :**

Dichlorhydrate de [αN-(carbéthoxy - 1 (S) phényl - 3 propyl) - (S) lysyl] - 2 carboxy - 3 (S) aza - 2 bicyclo [2,2,2] octane

En procédant, à partir du produit obtenu au stade prédédent, de la même façon qu'indique l'exemple 1 - Stade D, on obtient le dichlorhydrate de [αN-ß(carbéthoxy - 1 (S) phényl - 3 propyl) - (S) lysyl] - 2 carboxy - 3 (S) aza - 2 bicyclo [2,2,2] octane.

**EXEMPLE 3 : ETUDE PHARMACOLOGIQUE DU COMPOSE DE L'INVENTION POUR LEQUEL E = H**

Le composé selon l'invention pour lequel E = H a été testé par administration per os sur le chien éveillé.

La pression artérielle des chiens a été mesurée par un capteur de pression ("Statham P23 Db") après cathétérisation de l'aorte par l'intermédiaire de l'artère fémorale. L'enregistrement est réalisé par un appareil enregistreur ("Brush 400").

L'angiotensine I et l'angiotensine II sont injectées aux animaux par voie intra-veineuse respectivement à des doses de 0,5 et 0,10 γ/kg. On établit une courbe dose/activité pour chacune de ces hormones. On administre ensuite le composé selon l'invention pour lequel E = H par voie orale à la dose de 1 mg/kg. On établit ensuite une deuxième courbe dose/activité pour l'angiotensine I et pour l'angiotensine II après administration du produit essayé.

On constate que le composé de l'invention pour lequel E = H possède une activité rapide, intense et prolongée à une dose de 1 $mg.kg^{-1}$ et ceci lorsqu'il est administré par voie orale, ce qui le différencie nettement des composés de l'art intérieur. Un quart d'heure après administration d'un $mg.kg^{-1}$ du composé de l'invention per os chez le chien, le pourcentage d'inhibition de l'enzyme de conversion est de 80 %. Il est voisin de 100 %, 30 minutes après administration et demeure supérieur à 70 %, six heures après administration.

Comparativement aux dérivés du brevet européen 0 051 020, il apparaît que le résultat est surprenant lorsque l'on considère après administration d'une dose de 1$mg.kg^{-1}$ per os, la rapidité avec laquelle une intense activité est obtenue et la durée pendant laquelle cette activité est maintenue : une telle rapidité d'action n'avait été obtenue pour les dérivés du brevet européen n° 0 051 020 qu'après administration par voie intraveineuse, une durée d'action comparable n'avait pu être observée qu'après administration d'une dose nettement supérieure. La structure originale du composé de la présente invention est donc vectrice d'une activité très intéressante, d'un niveau surprenant par rapport aux composés de l'art antérieur.

**EXEMPLE 4 : COMPOSITION PHARMACEUTIQUE**

Comprimé : formule de préparation pour 1000 comprimés dosés à 2 mg de principe actif.
Dichlorhydrate de [αN-(carboxy - 1 (S) phényl - 3 propyl) - (S) - lysyl] - 2 carboxy - 3 (S) aza - 2 bicyclo [2,2,2] octane : 2 g

Hydroxy propyl cellulose :2 g
Amidon de blé :10 g
Lactose :100 g
Stéarate de magnesium :3 g
Talc :3 g

**Revendications pour les Etats contractants AT, BE, CH, DE, FR, GB, IT, LI, LU, NL, SE**

1/ Composés de formule (I) :

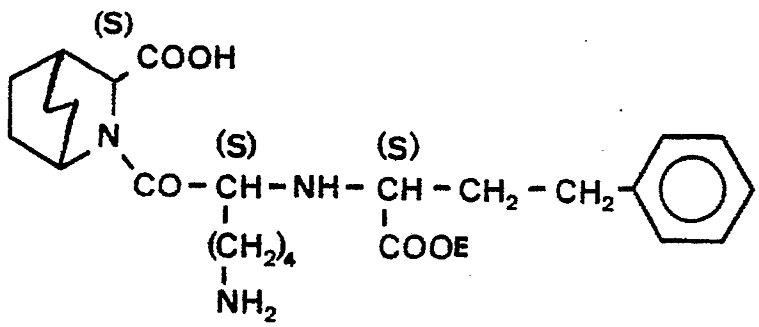

(I)

dans laquelle E est un atome d'hydrogène ou un radical alkyle inférieur ainsi que leurs sels d'addition à une base ou un acide pharmaceutiquement acceptables.

2/ Composé selon la revendication 1 dans lequel E signifie un atome d'hydrogène qui est l'[αN-(carboxy - 1 (S) - phényl - 3 propyl) (S) lysyl] - 2 carboxy - 3 (S) aza - 2 bicyclo [2,2,2] octane ainsi que ses sels d'addition à une base ou un acide pharmaceutiquement acceptables.

3/ Composé selon la revendication 1 dans lequel E signifie un groupement éthyle qui est l'[αN-(éthoxycarbonyl - 1 (S) - phényl - 3 propyl) - (S) lysyl] - 2 carboxy - 3 (S) aza - 2 bicyclo [2,2,2] octane ainsi que ses sels d'addition à une base ou un acide pharmaceutiquement acceptables.

4/ Procédé de préparation des composés de formule (I) caractérisé en ce que l'on soumet un dérivé de formule (III) :

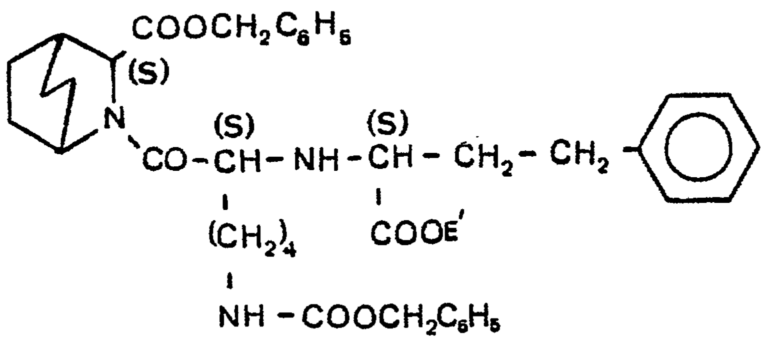

(III)

dans lequel E' représente un groupement alkyle inférieur ou benzyle
à hydrogénation en présence d'un catalyseur tel que le charbon palladié pour obtenir :
- le composé de formule (I) pour lequel E = H dans le cas où E' représente un groupement benzyle,
- un composé de formule (I) pour lequel E est un groupement alkyle inférieur dans le cas où E' représente un groupement alkyle inférieur, composé qui peut éventuellement être soumis à hydrolyse alcaline pour obtenir le composé de formule (I) pour lequel E = H,
le composé de formule (I) ainsi obtenu, si on le désire, pouvant être salifié par un acide ou une base minéral ou organique thérapeutiquement compatible ;
le composé de formule (III) étant lui-même obtenu :
- soit en soumettant à amination réductrice un oxo - 2 phényl - 4 butyrate de formule (IV) :

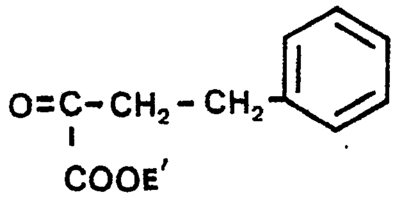

(IV)

où E' a la même signification que dans la formule (III), en présence d'un hydrure mixte de métal alcalin ou d'un cyanohydrure mixte de métal alcalin tel que le cyanoborohydrure de sodium, avec un dérivé de la lysine, dans lequel les groupements εaminé et carboxylique sont protégés par des groupements appropriés, tel que le εN benzyloxycarbonyl (S) lysinate de tert.butyle de formule (V) :

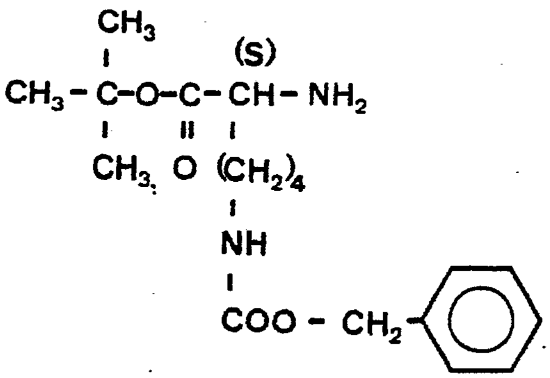

(V)

pour obtenir un dérivé de formule (VI) :

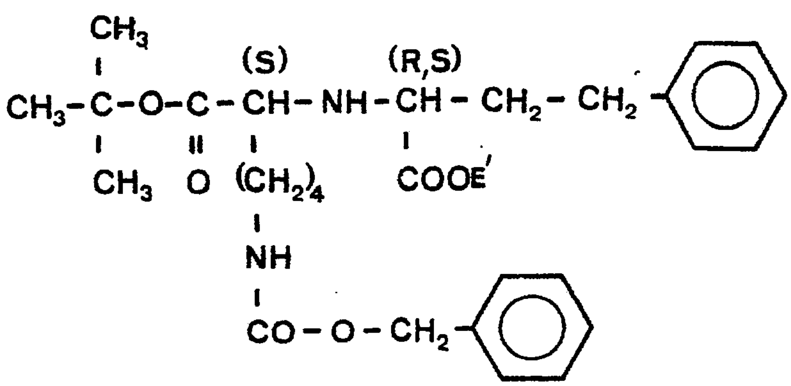

(VI)

dans lequel E' a la même signification que dans la formule (III), dont on isole les isomères (S, R) et (S, S) par une technique usuelle de séparation comme la chromatographie sur colonne de silice, l'isomère (S, S) ainsi obtenu étant alors soumis à une déprotection en milieu acide de la fonction acide carboxylique de la lysine pour obtenir un dérive de formule (VII) :

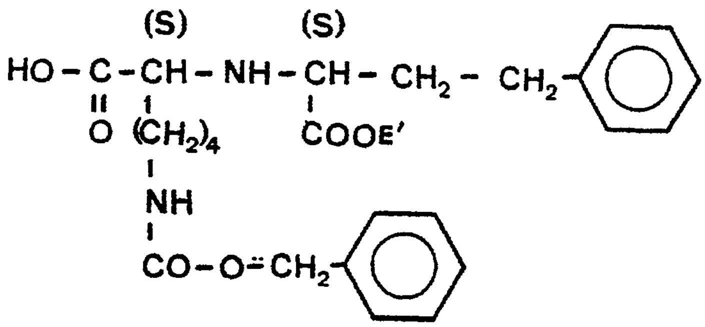

(VII)

dans lequel E' a la même signification que dans la formule (III),
qui est alors condensé avec un ester de l'acide aza - 2 bicyclooctane [2,2,2] carboxylique - 3 (S) et préférentiellement avec l'ester benzylique de formule (VIII) :

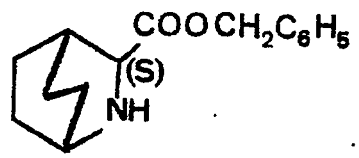

(VIII)

en présence d'un agent de couplage peptidique tel que le dicyclohexycarbodiimide en présence d'hydroxybenzotriazole,
pour obtenir un dérivé de formule (III),
- soit par couplage d'un ester de l'acide aza - 2 bicyclooctane [2,2,2] carboxylique - 3 (S) et préférentiellement de l'ester benzylique de formule ci-dessus référencée (VIII), avec un dérivé de la lysine dans lequel les groupements αet εaminés sont protégés par des groupements appropriés tel que la αN-tert.butoxycarbonyl εN-benzyloxycarbonyl (S) lysine de formule (IX) :

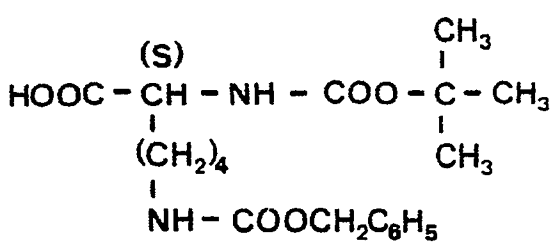

(IX)

en présence d'un agent de couplage peptidique tel que le dicyclohexylcarbodiimide en présence d'hydroxybenzotriazole,
pour obtenir un dérivé de formule (X) :

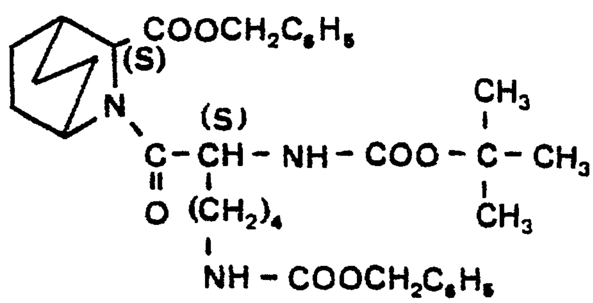

(X)

que l'on soumet à une déprotection en milieu acide de la fonction αaminée du reste lysyle,
pour obtenir un dérivé de formule (XI) :

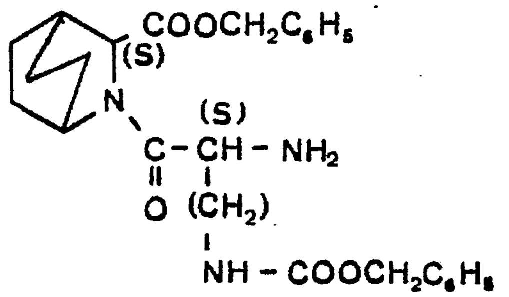

(XI)

que l'on soumet à amination réductrice en présence d'un hydrure mixte de métal alcalin ou d'un cyanohydrure mixte de métal alcalin tel que le cyanoborohydrure de sodium,
avec un dérivé de formule (IV) ci-dessus référencé,
pour obtenir le dérivé de formule (III) sous forme de racémique (III, R, S) :

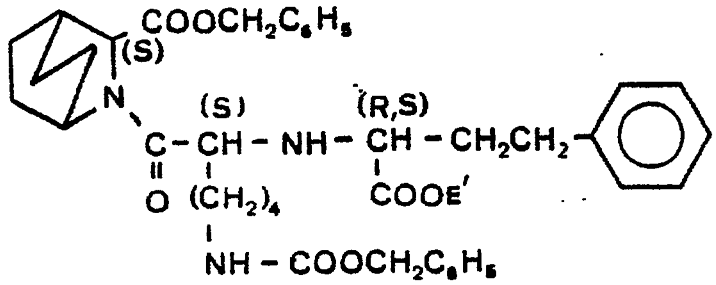

(III, R, S)

dans lequel E′ a la même signification que dans la formule (III),
dont on isole l'isomère (S, S, S) par une technique usuelle de séparation comme la chromatographie sur colonne de silice.

5/ Composés de structure (III) et (III, R, S) selon la revendication 4 ainsi que leurs sels d'addition à un acide minéral ou organique utiles pour la préparation des composés selon la revendication 1.

6/ Composition pharmaceutique contenant comme principe actif au moins un composé selon l'une des revendications 1 à 3 en combinaison avec un ou plusieurs excipients ou véhicules inertes non toxiques, pharmaceutiquement acceptables.

7/ Composition pharmaceutique contenant comme principe actif le composé selon la revendication 2 en combinaison avec un ou plusieurs excipients ou véhicules inertes non toxiques pharmaceutiquement acceptables.

8/ Composition pharmaceutique selon l'une quelconque des revendications 6 ou 7 contenant en outre, un ou plusieurs principes actifs choisis parmi diurétique, αadrénolytique, βbloquant, antagoniste calcique, agoniste des récepteurs dopaminergiques vasculaires.

**Revendications pour les Etats contractants ES, GR**

1/ Procédés de préparation des composés de formule (I) :

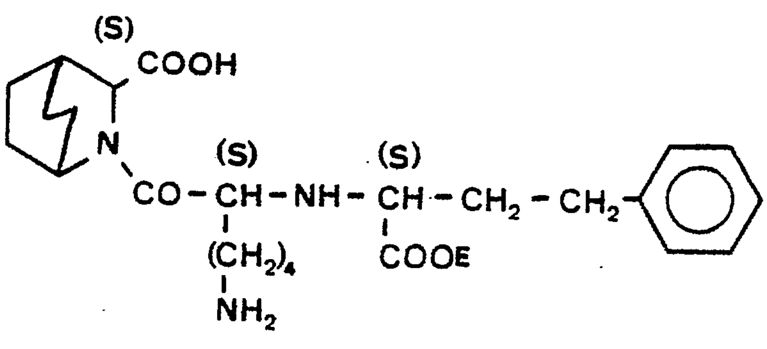

(I)

dans laquelle E est un atome d'hydrogène ou un radical alkyle inférieur ainsi que leurs sels d'addition à une base ou un acide pharmaceutiquement acceptables caractérisé en ce que l'on soumet un dérivé de formule (III) :

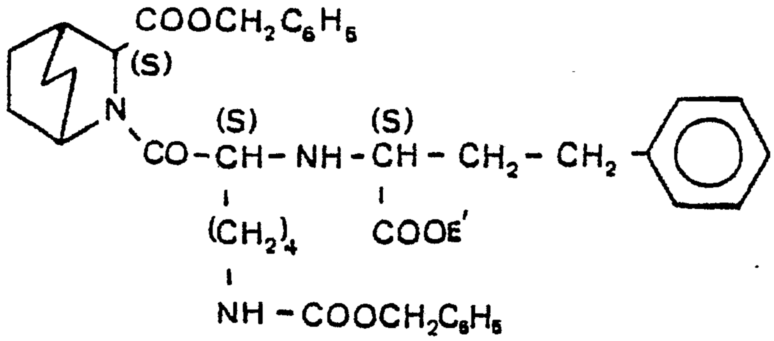

(III)

dans lequel E' représente un groupement alkyle inférieur ou benzyle
à hydrogénation en présence d'un catalyseur tel que le charbon palladié pour obtenir :
- le composé de formule (I) pour lequel E = H dans le cas où E' représente un groupement benzyle,
- un composé de formule (I) pour lequel E est un groupement alkyle inférieur dans le cas où E' représente un groupement alkyle inférieur, composé qui peut éventuellement être soumis à hydrolyse alcaline pour obtenir le composé de formule (I) pour lequel E = H,
le composé de formule (I) ainsi obtenu, si on le désire, pouvant être salifié par un acide ou une base minéral ou organique thérapeutiquement compatible ;
le composé de formule (III) étant lui-même obtenu :
- soit en soumettant à amination réductrice un oxo - 2 phényl - 4 butyrate de formule (IV) :

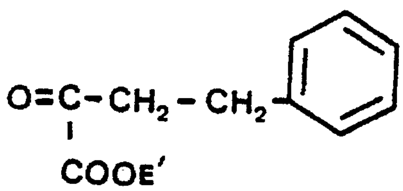

(IV)

où E' a la même signification que dans la formule (III),
en présence d'un hydrure mixte de métal alcalin ou d'un cyanohydrure mixte de métal alcalin tel que le cyanoborohydrure de sodium,
avec un dérivé de la lysine, dans lequel les groupements εaminé et carboxylique sont protégés par des groupements appropriés, tel que le εN-benzyloxycarbonyl (S) lysinate de tert.butyle de formule (V) :

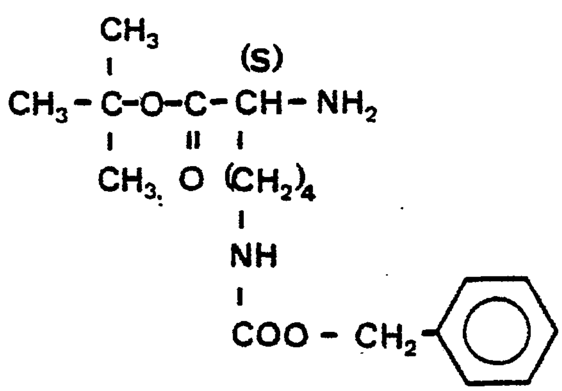

(V)

pour obtenir un dérivé de formule (VI) :

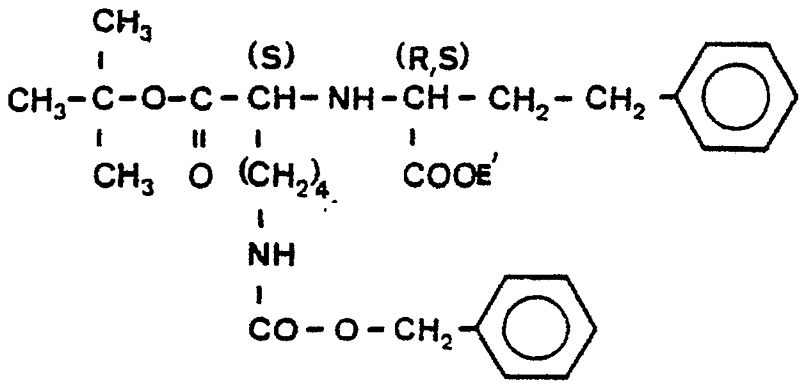

(VI)

dans lequel E' a la même signification que dans la formule (III), dont on isole les isomères (S, R) et (S, S) par une technique usuelle de séparation comme la chromatographie sur colonne de silice, l'isomère (S, S) ainsi obtenu étant alors soumis à une déprotection en milieu acide de la fonction acide carboxylique de la lysine pour obtenir un dérivé de formule (VII) :

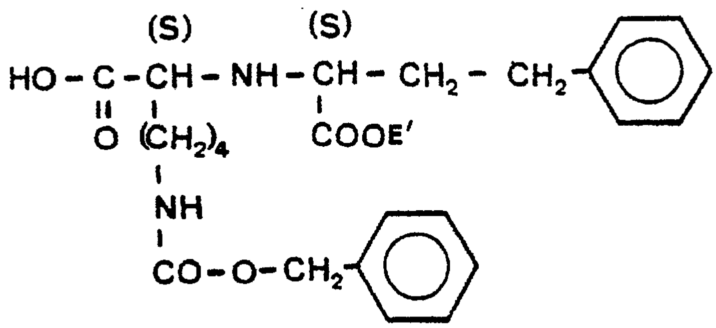

(VII)

dans lequel E' a la même signification que dans la formule (III), qui est alors condensé avec un ester de l'acide aza - 2 bicyclooctane [2,2,2] carboxylique - 3 (S) et préférentiellement avec l'ester benzylique de formule (VIII) :

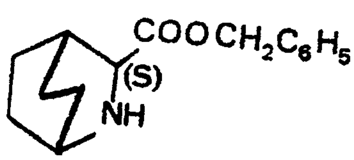

(VIII)

en présence d'un agent de couplage peptidique tel que le dicyclohexycarbodiimide en présence d'hydroxybenzotriazole, pour obtenir un dérivé de formule (III),
- soit par couplage d'un ester de l'acide aza - 2 bicyclooctane [2,2,2] carboxylique - 3 (S) et préférentiellement de l'ester benzylique de formule ci-dessus référencée (VIII), avec un dérivé de la lysine dans lequel les groupements αet εaminés sont protégés par des groupements appropriés tel que la αN-tert.butoxycarbonyl εN-benzyloxycarbonyl (S) lysine de formule (IX) :

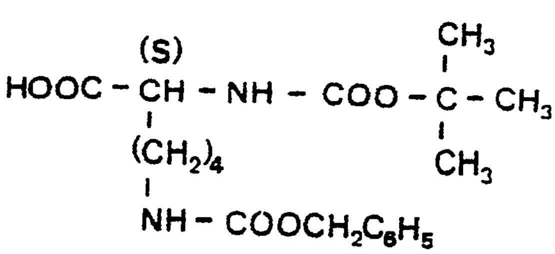

(IX)

en présence d'un agent de couplage peptidique tel que le dicyclohexylcarbodiimide en présence d'hydroxybenzotriazole,
pour obtenir un dérivé de formule (X) :

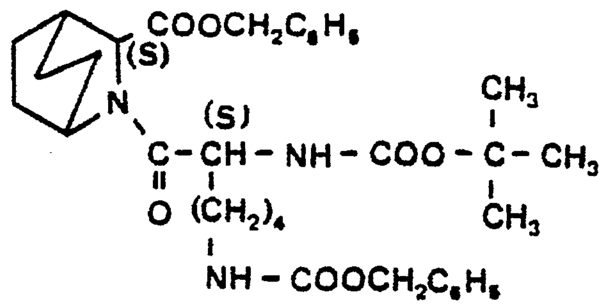

(X)

que l'on soumet à une déprotection en milieu acide de la fonction αaminée du rest lysyle,
pour obtenir un dérivé de formule (XI) :

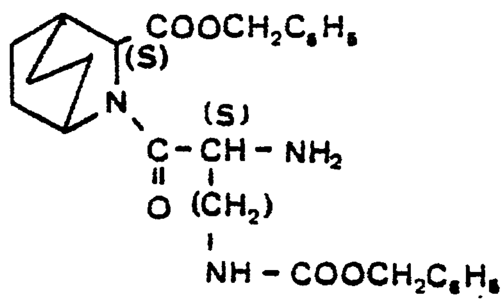

(XI)

que l'on soumet à amination réductrice en présence d'un hydrure mixte de métal alcalin ou d'un cyanohydrure mixte de métal alcalin tel que le cyanoborohydrure de sodium,
avec un dérivé de formule (IV) ci-dessus référence,
pour obtenir le dérivé de formule (III) sous forme de racémique (III, R, S) :

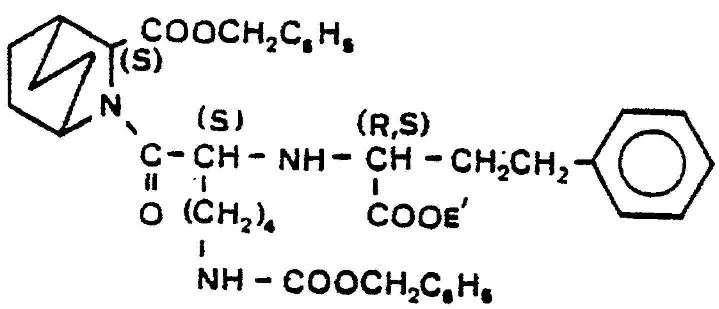

(III, R, S)

dans lequel E' a la même signification que dans la formule (III),
dont on isole l'isomère (S, S, S) par une technique usuelle de séparation comme la chromatographie sur colonne de silice.

2/ Procédés de préparation de composés de structure (III) et (III, R, S) selon la revendication 1, ainsi que leurs sels d'addition à un acide minéral ou organique utiles pour la préparation des composés selon la revendication 1.

3/ Procédés de préparation selon la revendication 1 du composé dans lequel E signifie un atome d'hydrogène qui est l'[αN-(carboxy - 1 (S) - phényl - 3 propyl) (S) lysyl] - 2 carboxy - 3 (S) aza - 2 bicyclo [2,2,2] octane ainsi que ses sels d'addition à une base ou un acide pharmaceutiquement acceptables.

4/ Procédés de préparation selon la revendication 1 du composé dans lequel E signifie un groupement éthyle qui est l'[αN-(éthoxycarbonyl - 1 (S) - phényl - 3 propyl) - (S) lysyl] - 2 carboxy - 3 (S) aza - 2 bicyclo [2,2,2] octane ainsi que ses sels d'addition à une base ou un acide pharmaceutiquement acceptables.

**Patentansprüche für die Vertragsstaaten ES, GR**

1. Verfahren zur Herstellung der Verbindung der Formel (I)

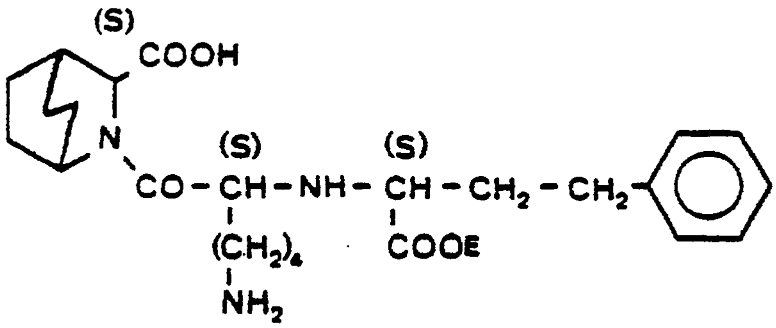

(I)

worin E ein Wasserstoffatom oder eine niedrigmolekulare Alkylgruppe bedeutet, sowie deren Additionssalzen mit einer pharmazeutisch annehmbaren Base oder Säure, dadurch gekennzeichnet, daß man ein Derivat der Formel (III)

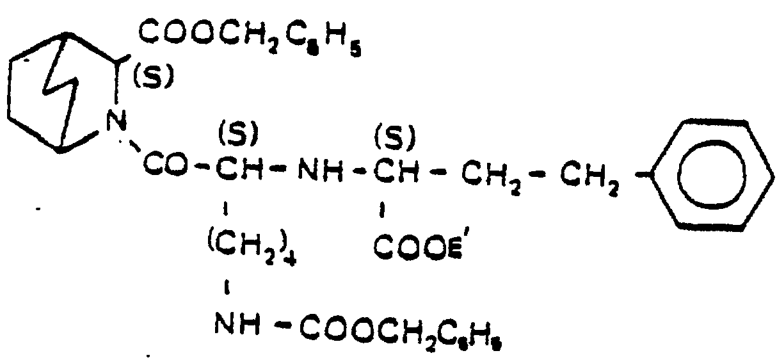

(III)

worin E' eine niedrigmolekulare Alkylgruppe oder eine Benzylgruppe bedeutet, in Gegenwart eines Katalysators, wie Palladium-auf-Kohlenstoff, hydriert zur Bildung:

– der Verbindung der Formel (I), worin E ein Wasserstoffatom bedeutet dann, wenn E' eine Benzylgruppe darstellt, oder

– einer Verbindung der Formel (I), in der E eine niedrigmolekulare Alkylgruppe bedeutet in dem Fall, da E' eine niedrigmolekulare Alkylgruppe darstellt, welche Verbindung gegebenenfalls einer alkalischen Hydrolyse unterworfen werden kann zur Bildung der Verbindung der Formel (I), worin E ein Wasserstoffatom bedeutet,

worauf man die erhaltene Verbindung der Formel (I) gewünschtenfalls mit eine therapeutisch verträglichen, anorganischen oder organischen Säure oder Base in ein Salz überführen kann; wobei die Verbindung der Formel (III) ihrerseits dadurch erhalten worden ist, daß man:

– entweder ein 2-Oxy-4-phenyl-butyrat der Formel (IV),

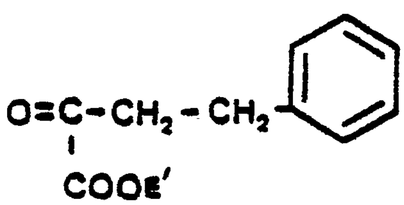

(IV)

worin E' die bezüglich der Formel (III) angegebenen Bedeutungen besitzt, in Gegenwart eines gemischten Alkalimetallhydrids oder eines gemischten Alkalimetallcyanohydrids, wie Natriumcyanoborhydrid, einer reduzierenden Aminierung mit einem Lysinderviat, worin die ε-Aminogruppen und Carboxylgruppen durch geeignete Gruppen geschützt sind, wie dem ε-N-Benzyloxycarbonyl-(S)-lysin-tert.-butylester der Formel (V)

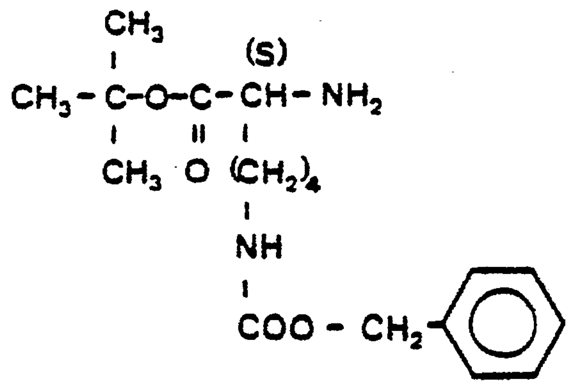

(V)

unterwirft, zur Bildung eines Derivats der Formel (VI)

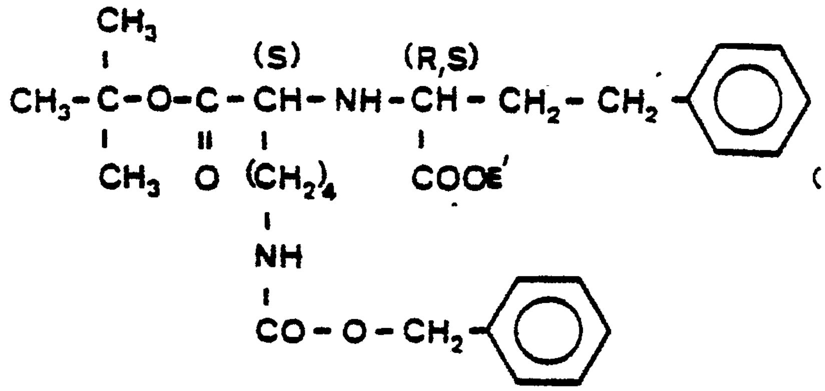

(VI)

worin E' die bezüglich der Formel (III) angegebenen Bedeutung besitzt, dessen Isomeren (S, R) und (S, S) man mit Hilfe üblicher Trennverfahren, wie der Chromatographie über eine mit Siliciumdioxid gefüllte Säure, isoliert, worauf man von dem in diese Weise erhaltenen (S, S)-Isomeren im sauren Medium die Schutzgruppen der Carbonsäuregruppe des Lysins abspaltet zur Bildung eines Derivates der Formel (VII)

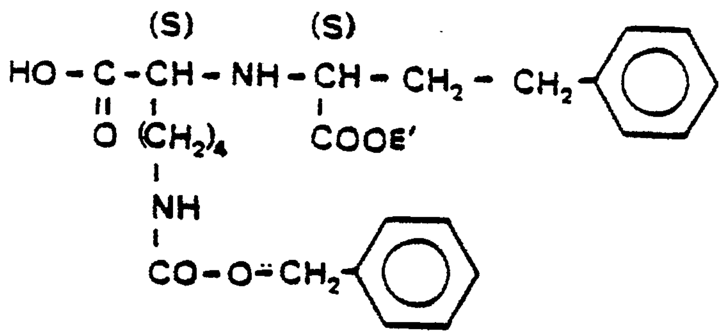

(VII)

worin E' die bezüglich der Formel (III) angegebenen Bedeutungen besitzt, welches man anschließend mit einem Ester der 2-Aza-bicyclo[2,2,2]octan-3(S)-carbonsäure, vorzugsweise mit dem Benzylester der Formel (VIII)

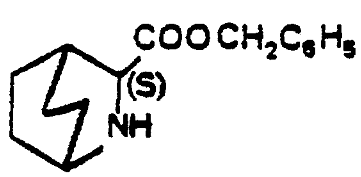

(VIII)

in Gegenwart eines Peptid-Kupplungsmittels, wie Dicyclohexylcarbodiimid, in Gegenwart von Hydroxybenzotriazol kondensiert zur Bildung eines Derivats der Formel (III),
– oder einen Ester der 2-Aza-bicyclo[2,2,2]octan-3(S)-carbonsäure und vorzugsweise den Benzylester der obigen Formel (VIII) in Gegenwart eines Peptidkupplungsmittels, wie Dicyclohexylcarbodiimid, in Gegenwart von Hydroxybenzotriazol mit einem Lysinderivat, dessen α- und ε-Aminogruppen durch geeignete Gruppen geschützt sind, wie α-N-tert.-Butoxycarbonyl-ε-N-benzyloxycarbonyl-(S)-lysin der Formel (IX)

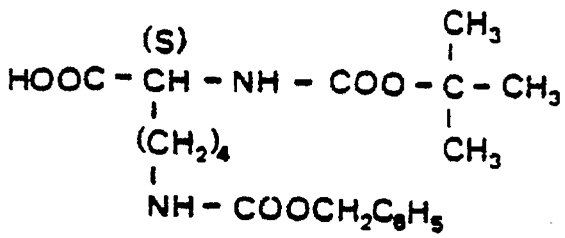

(IX)

kuppelt, so daß man ein Derivat der Formel (X)

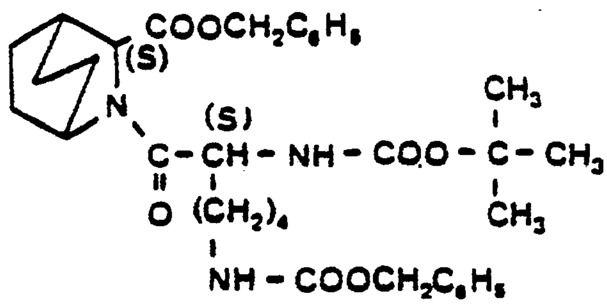

(X)

erhält, von dem man die Schutzgruppe der α-Aminofunktion des Lysylrestes in saurem Medium abspaltet zur Bildung eines Derivats der Formel (XI),

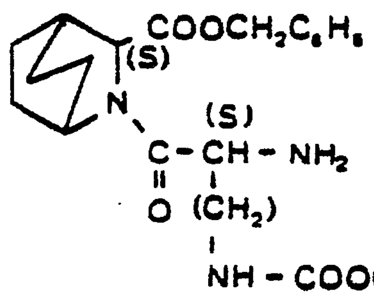

(XI)

welches man in Gegenwart eines gemischten Alkalimetallhydrids oder eines gemischten Alkalimetallcyanohydrids, wie Natriumcyanoborhydrid, mit einem Derivat der obigen Formel (IV) einer reduzierenden Aminierung unterwirft zur Bildung des Derivats der Formel (III) in Form des Racemats (III, R, S):

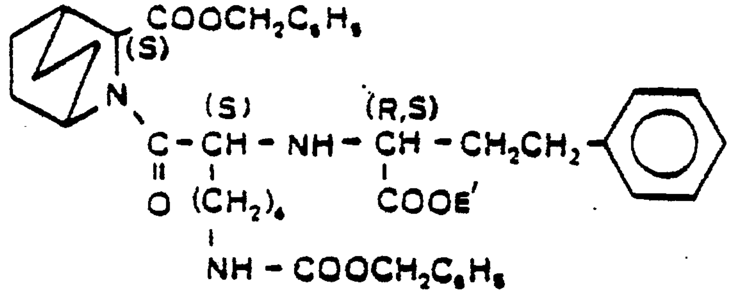

(III, R, S)

worin E′ die bezüglich der Formel (III) angegebenen Bedeutungen besitzt, dessen (S,S,S)-Isomeres man mit Hilfe einer an sich bekannteren Methode, wie der Chromatographie über eine mit Silicumdioxid gefüllte Säule, isoliert.

2. Verfahren zur Herstellung der Verbindung der Formeln (III) und (III, R, S) nach Anspruch 1, sowie ihrer Additionssalze mit einer anorganischen oder organischen Säure, die zur Herstellung der Verbindung nach Anspruch 1 verwendet werden können.

3. Verfahren nach Anspruch 1 zur Herstellung der Verbindung, worin E ein Wasserstoffatom bedeutet, nämlich 2-[α-N-(1(S)-Carboxycarbonyl-3-phenyl-propyl]-(S)-lysyl]-3(S)-carboxy-2-aza-bicyclo[2,2,2]octan, sowie ihrer Additionssalze mit einer pharmazeutisch annehmbaren Base oder Säure.

4. Verfahren nach Anspruch 1 zur Herstellung der Verbindung, worin E eine Ethylgruppe darstellt, nämlich 2-[α-N-(1(S)-Ethoxycarbonyl-3-phenyl-propyl)-(S)-lysyl]-3(S)-carboxy-2-aza-bicyclo[2,2,2] octan, sowie ihrer Additionssalze mit einer pharmazeutisch annehmbaren Base oder Säure.

**Patentansprüche für die Vertragsstaaten AT, BE, CH, DE, FR, GB, IT, LI, LU, NL, SE**

1. Verbindungen der Formel (I).

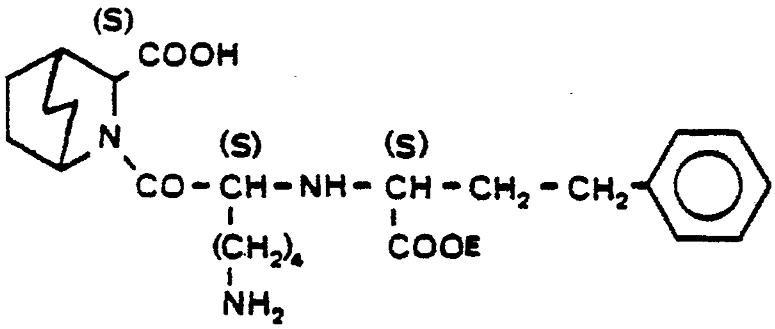

(I)

in der E ein Wasserstoffatom oder eine niedrigmolekulare Alkylgruppe bedeutet, sowie deren Additionssalze mit einer pharmazeutisch annehmbaren Base oder Säure.

2. Verbindung nach Anspruch 1, worin E ein Wasserstoffatom bedeutet, nämlich 2-[α-N-(1-(S)-Carboxy-3-phenyl-propyl)-(S)-lysyl]-3(S)-carboxy-2-aza-bicyclo[2,2,2]octan sowie dessen Additionssalze mit einer pharmazeutisch annehmbaren Base oder Säure.

3. Verbindung nach Anspruch 1, worin E eine Ethylgruppe bedeutet, nämlich 2-[α-N-(1(S)-Ethoxycarbonyl-3-phenyl-propyl)-(S)-lysyl]-3(S)-carboxy-2-aza-bicyclo[2,2,2]octan sowie dessen Additionssalze mit einer pharmazeutisch annehmbaren Base oder einer Säure.

4. Verfahren zur Herstellung der Verbindungen der Formel (I), dadurch gekennzeichnet, daß man ein Derivat der Formel (III)

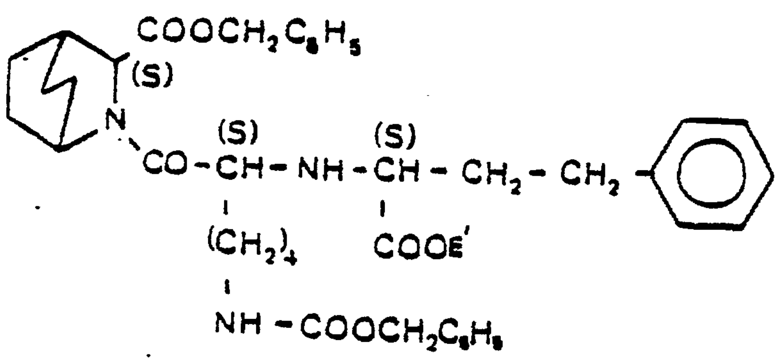

(III)

worin E′ eine niedrigmolekulare Alkylgruppe oder eine Benzylgruppe bedeutet, in Gegenwart eines Katalysators, wie Palladium-auf-Kohlenstoff, hydriert zur Bildung:

– der Verbindung der Formel (I), worin E ein Wasserstoffatom bedeutet dann, wenn E′ eine Benzylgruppe darstellt, oder

– einer Verbindung der Formel (I), in der E eine niedrigmolekulare Alkylgruppe bedeutet in dem Fall, da E′ eine niedrigmolekulare Alkylgruppe darstellt, welche Verbindung gegebenenfalls einer alkalischen Hydrolyse unterworfen werden kann zur Bildung der Verbindung der Formel (I), worin E ein Wasserstoffatom bedeutet,

worauf man die erhaltenen Verbindung der Formel (I) gewünschtenfalls mit einer therapeutisch verträglichen, anorganischen oder organischen Säure oder Base in ein Salz überführen kann; wobei die Verbindung der Formel (III) ihrerseits dadurch erhalten worden ist, daß man:

– entweder ein 2-Oxy-4-phenyl-butyrat der Formel (IV),

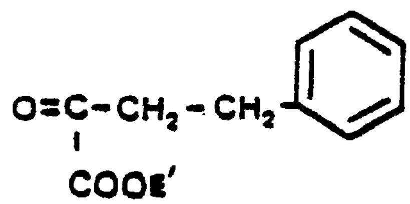

(IV)

worin E′ die bezüglich der Formel (III) angegebenen Bedeutungen besitzt, in Gegenwart eines gemischten Alkylimetallhydrids oder eines gemischten Alkalimetallcyanohydrids, wie Natriumcyanoborhydrid, einer reduzierenden Aminierung mit einem Lysinderivat, worin die ε-Aminogruppen und Carboxylgrukppen durch geeignete Gruppen geschützt sind, wie dem ε-N-Benzyloxycarbonyl-(S)-lysin-tert.-butylester der Formel (V)

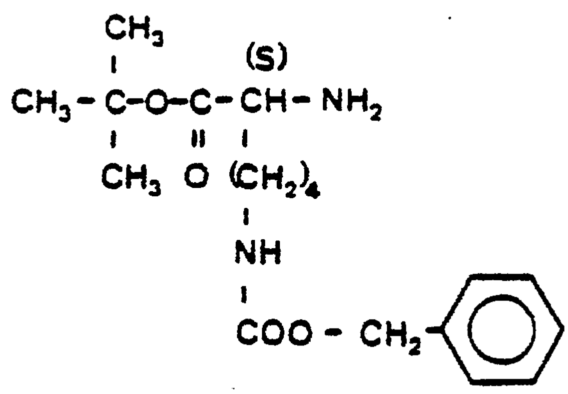

(V)

unterwirft, zur Bildung eines Derivats der Formel (VI)

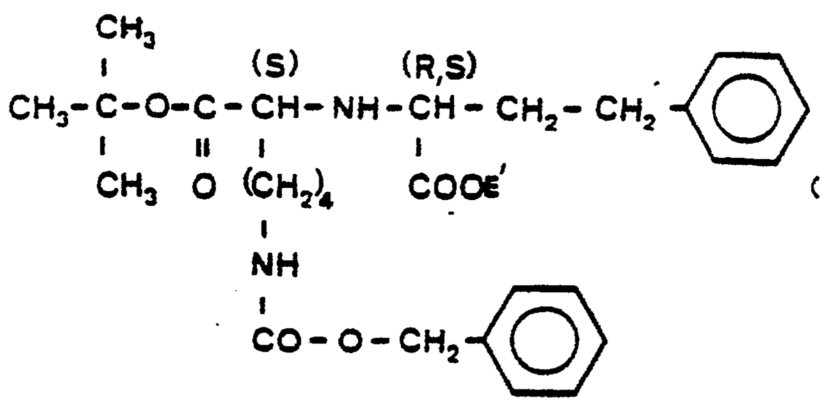

(VI)

worin E′ die bezüglich der Formel (III) angegebenen Bedeutungen besitzt, dessen Isomeren (S, R) und (S, S) man mit Hilfe üblicher Trennverfahren, wie der Chromatographie über eine mit Siliciumdioxid gefüllte Säure, isoliert, worauf man von dem in diese Weise erhaltenen (S, S)-Isomeren in sauren Medium die Schutzgruppen der Carbonsäuregruppe des Lysins abspaltet zur Bildung eines Derivates der Formel (VII)

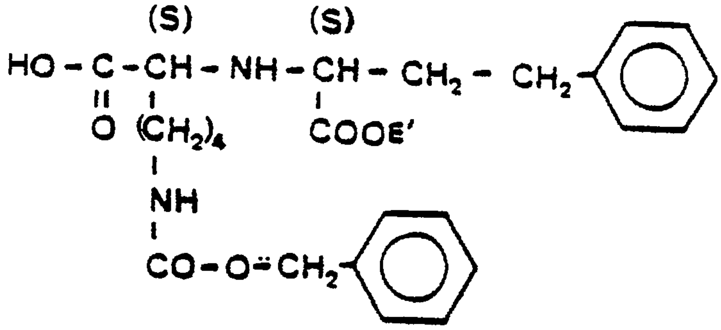

(VII)

worin E' die bezüglich der Formel (III) angegebenen Bedeutungen besitzt, welches man anschließend mit einem Ester der 2-Aza-bicyclo[2,2,2]octan-3(S)-carbonsäure, vorzugsweise mit dem Benzylester der Formel (VIII)

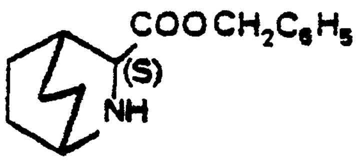

(VIII)

in Gegenwart eines Peptid-Kupplungsmittels, wie Dicyclohexylcarbodiimid, in Gegenwart von Hydroxybenzotriazol kondensiert zur Bildung eines Derivats der Formel (III).

– oder einen Ester der 2-Aza-bicyclo[2,2,2]octan-3(S)-carbonsäure und vorzugsweise den Benzylester der obigen Formel (VIII) in Gegenwart eines Peptidkupplungsmittels, wie Dicyclohexylcarbodiimid, in Gegenwart von Hydroxybenzotriazol mit einem Lysinderivat, dessen α- und ε-Aminogruppen durch geeignete Gruppen geschützt sind, wie α-N-tert.-Butoxycarbonyl-ε-N-benzyloxycarbonyl-(S)-lysin der Formel (IX)

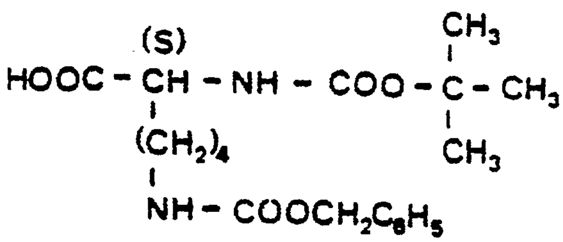

(IX)

kuppelt, so daß man ein Derivat der Formel (X)

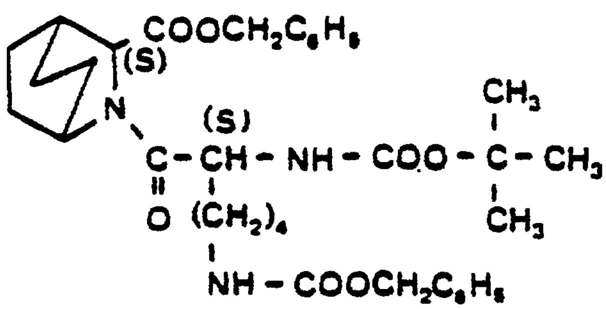

(X)

erhält, von dem man die Schutzgruppe der α-Aminofunktion des Lysylrestes in saurem Medium abspaltet zur Bildung eines Derivats der Formel (XI),

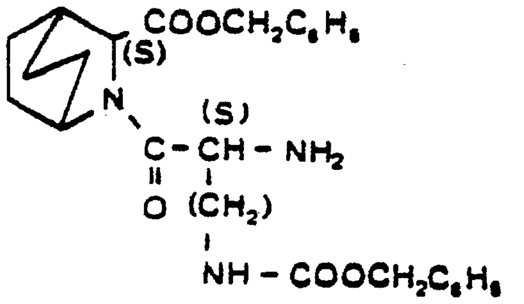

(XI)

welches man in Gegenwart eines gemischten Alkalimetallhydrids oder eines gemischten Alkalimetallcyanohydrids, wie Natriumcyanoborhydrid, mit einem Derivat der obigen Formel (IV) einer reduzierenden Aminierung unterwirft zur Bildung des Derivats der Formel (III) in Form des Racemats (III, R, S):

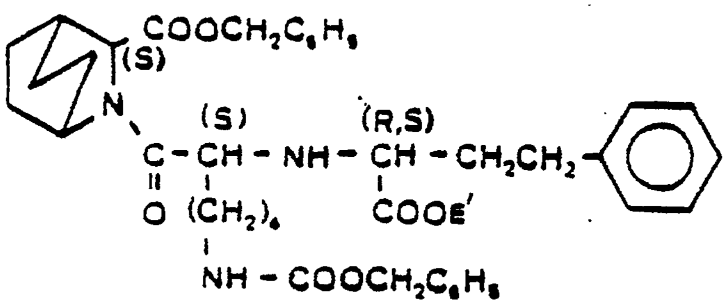

(III, R, S)

worin E′ die bezüglich der Formel (III) angegebenen Bedeutungen besitzt, dessen (S,S,S)-Isomeres man mit Hilfe einer an sich bekannteren Methode, wie der Chromatographie über eine mit Siliciumdioxid gefüllte Säule, isoliert.

5. Verbindungen der Formeln (III) und (III, R, S) nach Anspruch 4 sowie deren Additionssalze mit einer anorganischen oder organischen Säure, zur Verwendung für Herstellung der Verbindungen nach Anspruch 1.

6. Pharmazeutische Zubereitung enthaltend als Wirkstoff mindestens eine Verbindung nach einem der Ansprüche 1 bis 3 in Kombination mit einem oder mehreren pharmazeutisch annehmbaren, inerten, nicht-toxischen Trägermaterialien oder Bindemitteln.

7. Pharmazeutische Zubereitung enthaltend als Wirkstoff die Verbindung nach Anspruch 2 in Kombination mit einem oder mehreren pharmazeutisch annehmbaren, inerten, nicht-toxischen Trägermaterialien oder Bindemitteln.

8. Pharmazeutische Zubereitung nach einem der Ansprüche 6 oder 7, enthaltend zusätzlich einen oder mehrere Wirkstoffe ausgewählt aus den Diuretika, α-Adrenolytika, β-Blockern, Calcium-Antagonisten und Agonisten der vaskulären dopaminergen Rezeptoren.

**Claims for the contracting states ES, GR**

1. Process for preparing the compounds of formula (I):

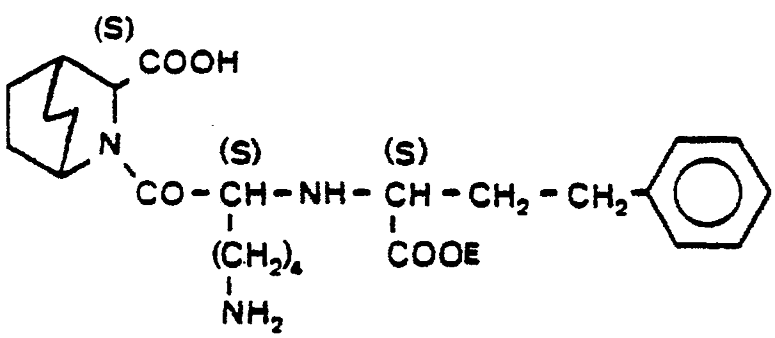

(I)

in which E is a hydrogen atom or a lower alkyl radical, as well as their addition salts with a pharmaceutically acceptable base or acid, characterised in that a derivative of formula (III):

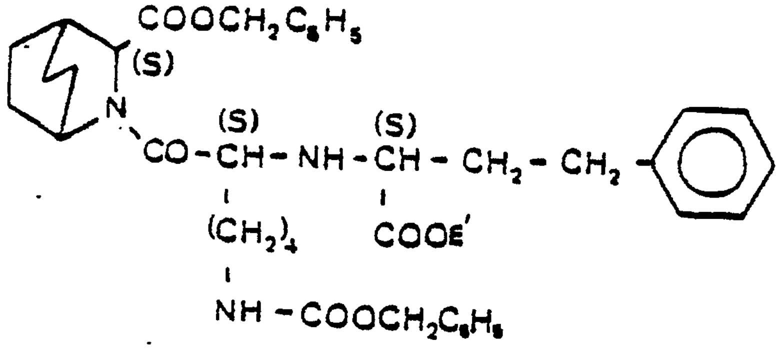

(III)

in which E′ denotes a lower alkyl or benzyl group, is subjected to hydrogenation in the presence of a catalyst such as palladinized charcoal, to obtain:

– the compound of formula (I) for which E = H, in the case where E′ denotes a benzyl group,

– a compound of formula (I) for which E is a lower alkyl group, in the case where E′ denotes a lower alkyl group, which compound can optionally be subjected to alkaline hydrolysis to obtain the compound of formula (I) for which E = H,

it being possible, if so desired, for the compound of formula (I) thereby obtained to be salified with a therapeutically compatible inorganic or organic acid or base; the compound of formula (III) itself being obtained:

– either by subjecting a 2-oxo-4-phenylbutyrate of formula (IV):

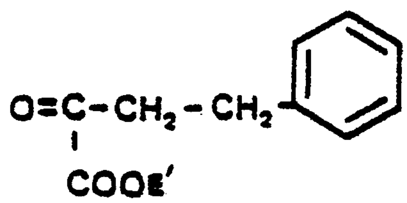

(IV)

or (sic) E′ has the same meaning as in the formula (III), to reductive amination in the presence of an alkali metal mixed hydride or an alkali metal mixed cyanohydride, such as sodium cyanoborohydride, with a lysine derivative in which the ε-amino and carboxyl groups are protected by suitable groups, such as tert-butyl (S)-N$^{\varepsilon}$-benzyloxycarbonyllysinate of formula (V):

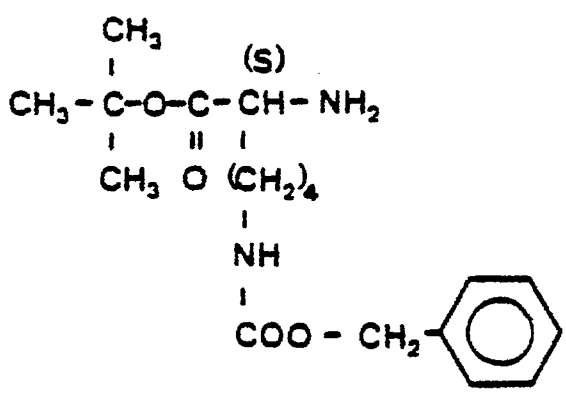

(V)

to obtain a derivative of formula (VI):

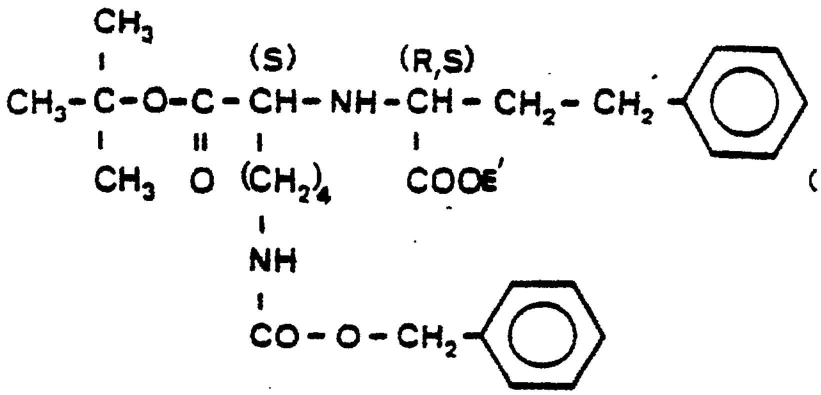

(VI)

in which E' has the same meaning as in the formula (III), the (S, R) and (S, S) isomers of which are isolated by a customary separation technique such as chromatography on a silica column, the (S, S) isomer thereby obtained then being subjected to a deprotection of the carboxylic acid group of the lysine in acid medium to obtain a derivative of formula (VII):

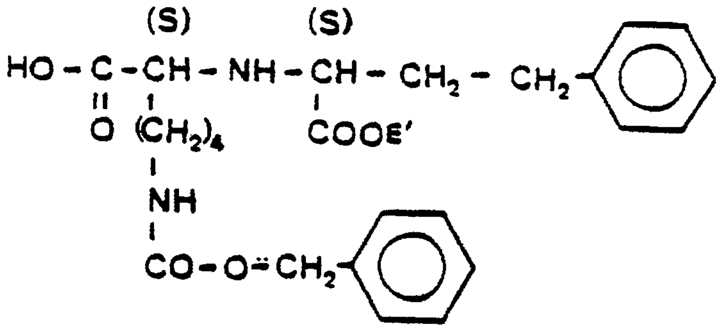

(VII)

in which E' has the meaning as in the formula (III), which is then condensed with a (3S)-2-azabicyclo[2.2.2]oc-tane-3-carboxylic acid ester, and preferably with the benzyl ester of formula (VIII):

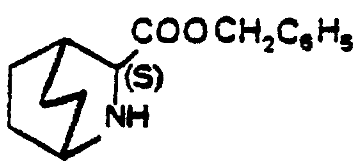

(VIII)

in the presence of a peptide coupling agent such as dicyclohexycarbodiimide (sic) in the presence of hydroxybenzotriazole, to obtain a derivative of formula (III),

– or by coupling a (3S)-2-azabicyclo[2.2.2]octane-3-carboxylic acid ester and preferably the benzyl ester of the above formula designated (VIII), with a lysine derivative in which the α- and ε-amino groups are protected by suitable groups, such as (S)-Nα-tert-butoxycarbonyl-Nε-benzyloxycarbonyllysine of formula (IX):

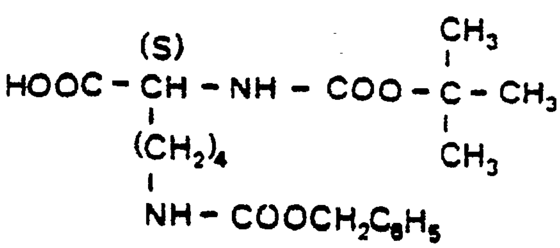

(IX)

in the presence of a peptide coupling agent such as dicyclohexylcarbodiimide in the presence of hydroxybenzotriazole, to obtain a derivative of formula (X):

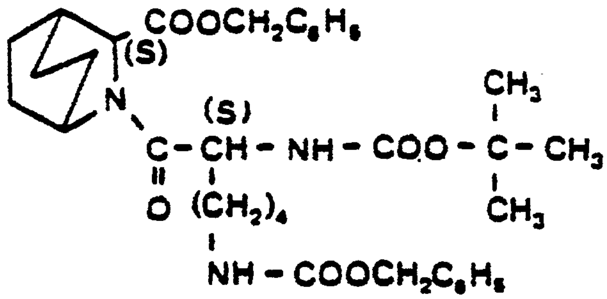

(X)

which is subjected to a deprotection of the α-amino group of the lysyl residue in acid medium, to obtain a derivative of formula (XI):

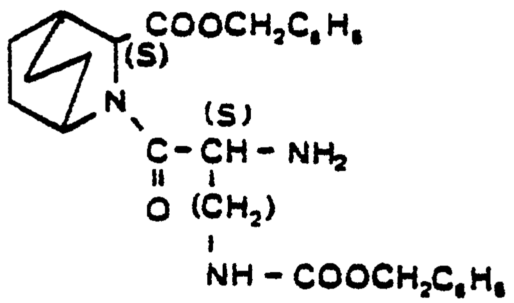

(XI)

which is subjected to reductive amination in the presence of an alkali metal mixed hydride or an alkali metal mixed cyanohydride, such as sodium cyanoborohydride, with a derivative of formula (IV) designated above, to obtain the derivative of formula (III) in racemic form (III, R, S):

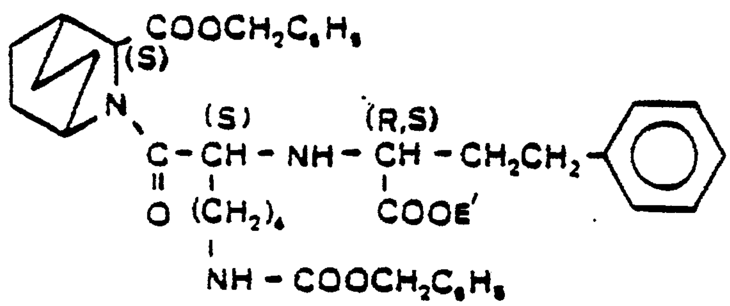

(III, R, S)

in which E′ has the same meaning as in the formular (III), the (S, S, S) isomer of which is isolated by a customary separation technique such as chromatography on a silica column.

2. Process for preparing compounds of structure (III) and (III, R, S) according to Claim 1, as well as their addition salts with an inorganic or organic acid, which are useful for the preparation of the compounds according to Claim 1.

3. Process for preparing, according to Claim 1, the compound in which E denotes a hydrogen atom, namely (3S)-2-{(S)-Nα-[(S)-1-carboxy-3-phenylpropyl]lysyl}-3-carboxy-2-azabicyclo-[2.2.2]octane, as well as its addition salts with a pharmaceutically acceptable base or acid.

4. Process for preparing, according to Claim 1, the compound in which E denotes an ethyl group, namely (3S)-2-{(S)-Nα-[(S)-1ethoxycarbonyl-3-phenylpropyl]lysyl}-3-carboxy-2-azabicyclo[2.2.2.]octane, as well as its addition salts with a pharmaceutically acceptable base or acid.

**Claims for the contracting states AT, BE, CH, DE, FR, GB, IT, LI, LU, NL, SE**

1. Compounds of formula (I):

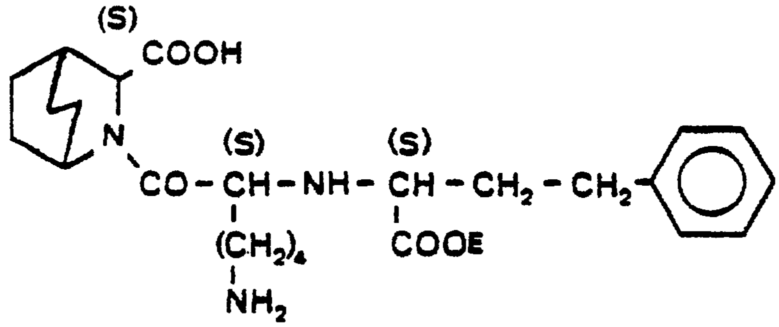

(I)

in which E is a hydrogen atom or a lower alkyl radical, as well as their addition salts with a pharmaceutically acceptable base or acid.

2. Compound according to Claim 1 in which E denotes a hydrogen atom, namely (3S)-2-{(S)-Nα-[(S)-1-carboxy-3-phenylpropyl]lysyl}-3-carboxy-2-azabicyclo-[2.2.2]octane, as well as its addition salts with a pharmaceutically acceptable base or acid.

3. Compound according to Claim 1 in which E denotes an ethyl group, namely (3S)-2-{(S)-Nα-[(S)-1-ethoxycarbonyl-3-phenylpropyl]lysyl}-3-carboxy-2-azabicyclo[2.2.2]octane, as well as its addition salts with a pharmaceutically acceptable base or acid.

4. Process for preparing the compounds of formula (I), characterized in that a derivative of formula (III):

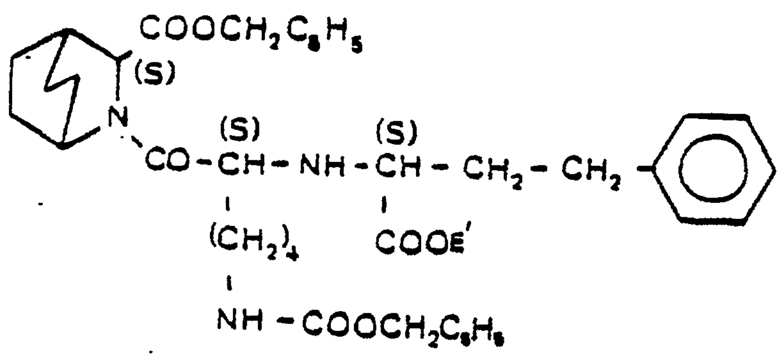

(III)

in which E′ denotes a lower alkyl or benzyl group, is subjected to hydrogenation in the presence of a catalyst such as palladinized charcoal, to obtain:

– the compound of formula (I) for which E=H, in the case where E′ denotes a benzyl group,

– a compound of formula (I) for which E is a lower alkyl group, in the case where E′ denotes a lower alkyl group, which compound can optionally be subjected to alkaline hydrolysis to obtain the compound of formula (I) for which E=H,

it being possible, if so desired, for the compound of formula (I) thereby obtained to be salified with a therapeutically compatible inorganic or organic acid or base; the compound of formula (III) itself being obtained:

– either by subjecting a 2-oxo-4-phenylbutyrate of formula (IV):

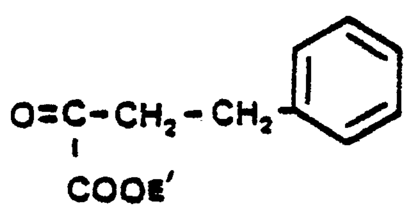

(IV)

where E′ has the same meaning as in the formula (III), to reductive amination in the rpesence of an alkali metal mixed hydride or an alkali metal mixed cyanohydride, such as sodium cyanoborohydride,

with a lysine derivative in which the ε-amino and carboxyl groups are protected by suitable groups, such as tert-butyl (S)-Nε-benzyloxycarbonyllysinate of formula (V):

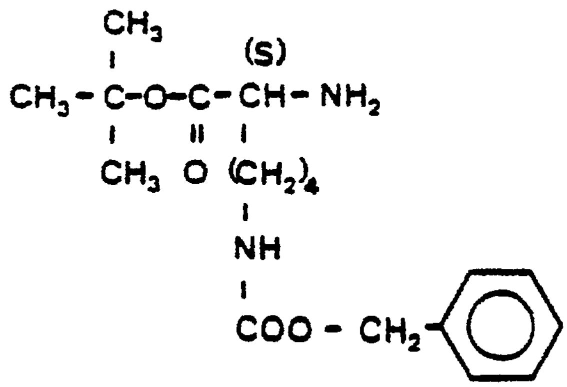

(V)

to obtain a derivative of formula (VI):

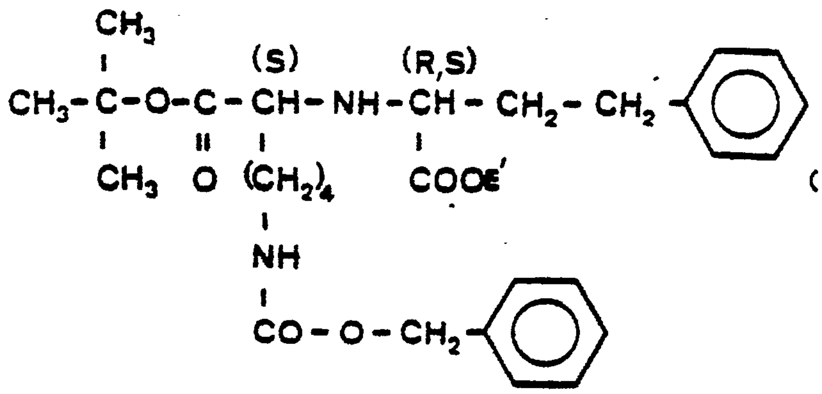

(VI)

in which E' has the same meaning as in the formula (III), the (S, R) and (S, S) isomers of which are isolated by a customary separation technique such as chromatography on a silica column, the (S, S) isomer thereby obtained then being subjected to a deprotection of the carboxylic acid group of the lysine in acid medium to obtain a derivative of formula (VII):

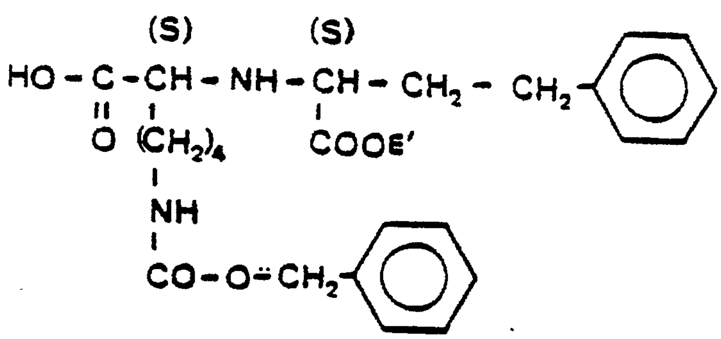

(VII)

in which E' has the meaning as in the formula (III), which is then condensed with a (3S)-2-azabicyclo [2.2.2]octane-3-carboxylic acid ester, and preferably with the benzyl ester of formula (VIII):

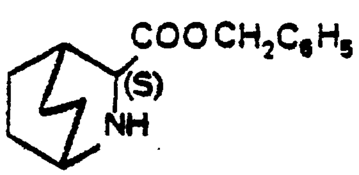

(VIII)

in the presence of a peptide coupling agent such as dicyclohexycarbodiimide (sic) in the presence of hydroxybenzotriazole, to obtain a derivative of formula (III),

– or by coupling a (3S)-2-azabicyclo[2.2.2]octane-3-carboxylic acid ester and preferably the benzyl ester of the above formula designated (VIII), with a lysine derivative in which the α- and ε-amino groups are protected by suitable groups, such as (S)-Nα-tert-butoxycarbonyl-Nε-benzyloxycarbonyllysine of formula (IX):

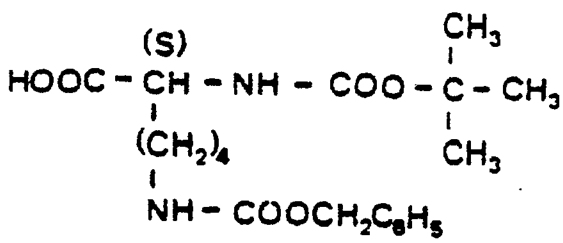

(IX)

in the presence of a peptide coupling agent such as dicyclohexylcarbodiimide in the presence of hydroxybenzotriazole, to obtain a derivative of formula (X):

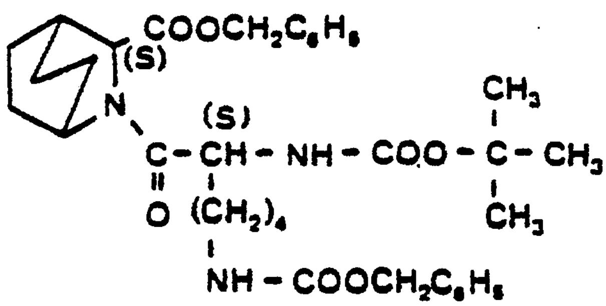

(X)

which is subjected to a deprotection of the α-amino group of the lysyl residue in acid medium, to obtain a derivative of formula (XI):

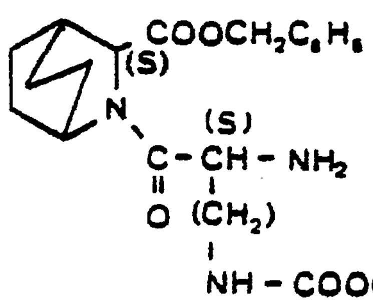

(XI)

which is subjected to reductive amination in the presence of an alkali metal mixed hydride or an alkali metal mixed cyanohydride, such as sodium cyanoborohydride, with a derivative of formula (IV) designated above, to obtain the derivative of formula (III) in racemic form (III, R, S):

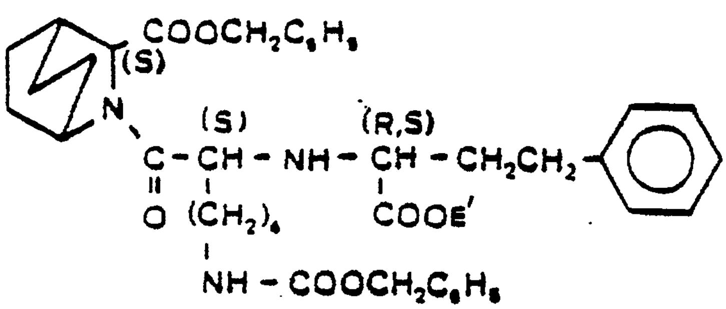

(III, R, S)

in which E' has the same meaning as in the formula (III), the (S, S, S) isomer of which is isolated by a customary separation technique such as chromatography on a silica solumn.

5. Compounds of structure (III) and (III, R, S) according to Claim 4, as well as their addition salts with an inorganic or organic acid, which are useful for the preparation of the compounds according to Claim 1.

6. Pharmaceutical composition containing, as active principle, at least one compound according to one of Claims 1 to 3, in combination with one or more pharmaceutically acceptable, non-toxic inert vehicles or excipients.

7. Pharmaceutical composition containing, as active principle, the compound according to Claim 2, in combination with one or more pharmaceutically acceptable, non-toxic inert vehicles or excipients.

8. Pharmaceutical composition according to either of Claims 6 and 7, containing, in addition, one or more active principles chosen from a diuretic, α-adrenolytic, β-blocker, calcium antagonist and vascular dopaminergic receptor agonist.